(19) **Europäisches Patentamt / European Patent Office / Office européen des brevets**

(11) **EP 4 599 761 A1**

(12) **EUROPEAN PATENT APPLICATION**
published in accordance with Art. 153(4) EPC

(43) Date of publication:
**13.08.2025   Bulletin 2025/33**

(21) Application number: **24806606.0**

(22) Date of filing: **15.05.2024**

(51) International Patent Classification (IPC):
***A61B 5/091*** (2006.01)

(52) Cooperative Patent Classification (CPC):
**A61B 5/0205; A61B 5/087; A61B 5/091;**
**A61B 5/145; A61B 7/00**

(86) International application number:
**PCT/CN2024/093354**

(87) International publication number:
**WO 2024/235256 (21.11.2024 Gazette 2024/47)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **18.05.2023   CN 202310567670**

(71) Applicant: **Huawei Technologies Co., Ltd.**
**Shenzhen, Guangdong 518129 (CN)**

(72) Inventors:
• **LI, Jing**
**Shenzhen, Guangdong 518129 (CN)**
• **CUI, Yuqi**
**Shenzhen, Guangdong 518129 (CN)**
• **CHEN, Wenjuan**
**Shenzhen, Guangdong 518129 (CN)**

(74) Representative: **Goddar, Heinz J.**
**Boehmert & Boehmert**
**Anwaltspartnerschaft mbB**
**Pettenkoferstrasse 22**
**80336 München (DE)**

(54) **HEALTH DATA DETECTION SYSTEM AND METHOD, AND DEVICE**

(57)   This application provides a health data detection system and method, and a device. An electronic device receives audio data and physiological data that are sent by a wearable device. The electronic device determines pulmonary function detection data based on the audio data and the physiological data. The electronic device displays a first graph. The first graph includes a first standard curve corresponding to first vital capacity standard data and a first graphical marker corresponding to the pulmonary function detection data. According to the method, the electronic device may test a pulmonary function of a user in collaboration with the wearable device, to obtain pulmonary function detection data of the user and display the pulmonary function detection data, thereby implementing convenience of a pulmonary function test. In addition, the electronic device may further compare, for display, an actual value and a theoretical value of a detected pulmonary function index in a graph, so that the user can intuitively observe whether the detected pulmonary function index falls within a normal range.

FIG. 4L

**Description**

This application claims priority to Chinese Patent Application No. 202310567670.0, filed with the China National Intellectual Property Administration on May 18, 2023 and entitled "HEALTH DATA DETECTION SYSTEM AND METHOD, AND DEVICE", which is incorporated herein by reference in its entirety.

**TECHNICAL FIELD**

This application relates to the field of terminal technologies, and in particular, to a health data detection system and method, and a device.

**BACKGROUND**

Breathing is one of the most important behaviors in human daily activities. With an increase in work pressure and impact of living habits, more users suffer from a respiratory disease. A pulmonary function test is an important means of detecting the respiratory disease. At present, a device for the pulmonary function test is inconvenient, and needs to be operated under professional guidance, which is time-consuming and costly. Consequently, a pulmonary function test rate is low, and a large quantity of respiratory diseases are not diagnosed. How to conveniently detect a pulmonary function of a user is to be further studied.

**SUMMARY**

This application provides a health data detection system and method, and a device. An electronic device may test a pulmonary function of a user in collaboration with a wearable device, to obtain pulmonary function detection data of the user and display the pulmonary function detection data, thereby implementing convenience of a pulmonary function test.

According to a first aspect, this application provides a health data display method. The method includes: An electronic device receives audio data and physiological data that are sent by a wearable device. The audio data is collected by the wearable device by using a microphone, and the physiological data is collected by the wearable device by using one or more sensors. The electronic device determines pulmonary function detection data based on the audio data and the physiological data. The electronic device displays a first graph. The first graph includes a first standard curve corresponding to first vital capacity standard data and a first graphical marker corresponding to the pulmonary function detection data.

The first vital capacity standard data may be pre-stored in the device by the electronic device, or the first vital capacity standard data may be obtained by the electronic device from a server in real time or obtained from another device.

The pulmonary function detection data includes any one or more of the following: a vital capacity, a forced expiratory volume in 1 second/forced vital capacity ratio, a forced expiratory volume in 1 second, peak expiratory flow, peak inspiratory flow, maximal expiratory pressure, maximal inspiratory pressure, maximal voluntary ventilation, and maximal mid-expiratory flow.

The forced expiratory volume in 1 second/forced vital capacity ratio indicates a percentage of an amount of air exhaled in a first second to a total amount of air exhaled in effective duration.

The forced expiratory volume in 1 second indicates a total amount of air exhaled in the first second.

The peak expiratory flow indicates an instantaneous maximum value of exhaled air flow in a maximum forced exhalation process.

The peak inspiratory flow indicates an instantaneous maximum value of inhaled air flow in a maximum forced inhalation process.

The maximal expiratory pressure indicates a maximum value of oral pressure capable of being generated in the maximum forced exhalation process.

The maximal inspiratory pressure indicates a maximum value of oral pressure capable of being generated in the maximum forced inhalation process.

The maximal voluntary ventilation indicates maximum ventilation in 1 minute.

The maximal mid-expiratory flow indicates a mean expiratory flow rate when 25% to 75% of the vital capacity is exhaled forcibly.

According to the method, the electronic device may test a pulmonary function of a user in collaboration with the wearable device, to obtain pulmonary function detection data of the user and display the pulmonary function detection data, thereby implementing convenience of a pulmonary function test. In addition, the electronic device may further compare, for display, an actual value and a theoretical value of a detected pulmonary function index in a graph, so that the user can intuitively observe whether the detected pulmonary function index falls within a normal range.

According to the first aspect, in a possible implementation, the first vital capacity standard data is related to a first gender of a first user and a first height of the first user, and the first standard curve includes a first curve, a second curve, and a third curve; the first curve includes a curve of a change in vital capacity data of a user group of the first gender at the first height with an age; the second curve includes a curve of a change in vital capacity data of the user group of the first gender at a second height with the age; the third curve includes a curve of a change in vital capacity data of the user group of the first gender at a third height with the age; and the second height is greater than the first height, and the third height is less than the first height.

Herein, the first vital capacity standard data is related to a gender and a height of the detected user (the first user). Users of different genders and different heights correspond to different first vital capacity standard data.

According to the first aspect, in a possible implementation, the second height includes an average minimum height of the user group of the first gender, and the third height is an average maximum height of the user group of the first gender.

Herein, a generation manner of obtaining the second curve and the third curve is shown. The generation manner of the second curve and the third curve is unrelated to the first height of the first user, and is related to the first gender of the first user.

According to the first aspect, in a possible implementation, a difference between the first height and the second height is a first threshold, and a difference between the third height and the first height is a second threshold; and the first threshold is the same as or different from the second threshold.

Herein, another generation manner of obtaining the second curve and the third curve is shown. The generation manner of the second curve and the third curve is related to the first height of the first user.

According to the first aspect, in a possible implementation, after the electronic device displays the first graph, the method further includes: The electronic device receives a first operation performed by a user. The electronic device obtains second vital capacity standard data in response to the first operation. The electronic device displays a second graph. The second graph includes a second standard curve corresponding to the second vital capacity standard data and a second graphical marker corresponding to the pulmonary function detection data, and the second vital capacity standard data is related to the first gender of the first user. The second standard curve includes a plurality of curves of changes in vital capacity data of the user group of the first gender at different heights with the age.

The second vital capacity standard data is different from the first vital capacity standard data. The second vital capacity standard data may be pre-stored in the device by the electronic device, or the second vital capacity standard data may be obtained by the electronic device from the server in real time or obtained from another device.

The electronic device may display the second vital capacity standard data by using a plurality of curves. The plurality of curves may be related to the first gender of the first user, and unrelated to the first height of the first user.

In another embodiment, before the electronic device displays the pulmonary function detection data, the electronic device may also notify the user to select one piece of pulmonary function standard data from a plurality of pieces of pulmonary function standard data, and compare, for display, the pulmonary function standard data selected by the user and the pulmonary function detection data.

In this implementation, the electronic device may receive a user operation to obtain different pulmonary function standard data, and compare the pulmonary function detection data and the different pulmonary function standard data for display. That is, the pulmonary function detection data of the detected user is displayed by switching graphs of different styles.

According to the first aspect, in a possible implementation, before the electronic device displays the first graph, the method further includes: The electronic device displays a first user interface. The first user interface further includes first prompt information, the first prompt information includes identifiers of a plurality of users, and the identifiers of the plurality of users include an identifier of the first user. The electronic device receives a first selection operation performed by the user on the identifier of the first user in the first user interface. The electronic device obtains the first vital capacity standard data after receiving the selection operation performed on the identifier of the first user.

In this implementation, the electronic device may detect pulmonary functions of different users, for example, detect pulmonary functions of different family members. The first graph is related to the gender and the height of the detected user or the gender of the detected user. If different detected users have different genders and heights or different detected users have different genders, the electronic device also obtains different first standard curves. Therefore, before displaying the first graph, the electronic device may notify the user to select or add the detected user, to determine the gender and the height of the detected user or the gender of the detected user.

Optionally, if the electronic device does not store a gender and a height of a currently detected user or a gender of a currently detected user, the electronic device may further notify the user to enter the gender and the height of the currently detected user or the gender of the currently detected user.

According to the first aspect, in a possible implementation, after the electronic device determines the pulmonary function detection data based on the audio data and the physiological data, the method further includes: The electronic device stores the pulmonary function detection data in a storage area corresponding to the first user. Different users correspond to different storage areas.

In this implementation, pulmonary function detection data of different detected users is stored in different storage areas, to facilitate subsequent obtaining and viewing of the pulmonary function detection data of the different users.

According to the first aspect, in a possible implementation, the first user interface further includes a first control, and before the electronic device receives audio data and physiological data that are sent by a wearable device, the method further includes: The electronic device receives a second operation performed by the user on the first control. The electronic device sends a first message to the wearable device in response to the second operation. The first message indicates the wearable device to collect the audio data and the physiological data.

In this way, before starting to detect the pulmonary function, the electronic device may send a message to the wearable device after receiving a detection start operation of the user, so that the wearable device starts to collect audio data and physiological data of the detected user after receiving the message sent by the electronic device.

According to the first aspect, in a possible implementation, after the electronic device displays the first graph, the method further includes: The electronic device displays a second user interface. A second control is displayed in the second user interface. The electronic device receives a third operation performed by the user on the second control. The electronic device displays a third graph in response to the third operation. The third graph includes pulmonary function detection data in a first time period.

In this way, the electronic device may store pulmonary function detection data of the detected user in specific duration, and the electronic device may also receive a user operation to view the pulmonary function detection data of the detected user in the specific duration.

According to the first aspect, in a possible implementation, the second user interface further includes second prompt information, the second prompt information includes the identifiers of the plurality of users, and the identifiers of the plurality of users include the identifier of the first user; and before the electronic device displays the third graph, the method further includes: The electronic device receives a second selection operation performed by the user on the identifier of the first user in the second user interface. The electronic device obtains pulmonary function detection data of the first user in the first time period based on the identifier of the first user in response to the second selection operation.

The electronic device stores pulmonary function detection data of different users in specific duration. When the electronic device receives a user operation to view the pulmonary function detection data of the user in the specific duration, the electronic device may notify the user to enter an identifier of the detected user, and obtain the pulmonary function detection data of the detected user in the specific duration from pulmonary function detection data of a plurality of detected users based on the identifier that is of the detected user and that is entered by the user.

According to the first aspect, in a possible implementation, the audio data includes any one or more of the following: a cough sound, a blow sound, and a speaking sound; and the physiological data includes any one or more of the following: a heart rate, heart rate variability, a respiratory rate, and blood oxygen.

According to a second aspect, this application provides a health data display system. The system includes a wearable device and an electronic device, the wearable device establishes a communication connection to the electronic device, and the wearable device includes a microphone and one or more motion sensors. The wearable device is configured to: collect audio data by using the microphone, and collect physiological data by using the one or more sensors. The wearable device is further configured to send the audio data and the physiological data to the electronic device. The electronic device is configured to determine pulmonary function detection data based on the audio data and the physiological data. The electronic device is further configured to display a first graph. The first graph includes a first standard curve corresponding to first vital capacity standard data and a first graphical marker corresponding to the pulmonary function detection data.

The pulmonary function detection data includes any one or more of the following: a vital capacity, a forced expiratory volume in 1 second/forced vital capacity ratio, a forced expiratory volume in 1 second, peak expiratory flow, peak inspiratory flow, maximal expiratory pressure, maximal inspiratory pressure, maximal voluntary ventilation, and maximal mid-expiratory flow.

The forced expiratory volume in 1 second/forced vital capacity ratio indicates a percentage of an amount of air exhaled in a first second to a total amount of air exhaled in effective duration.

The forced expiratory volume in 1 second indicates a total amount of air exhaled in the first second.

The peak expiratory flow indicates an instantaneous maximum value of exhaled air flow in a maximum forced exhalation process.

The peak inspiratory flow indicates an instantaneous maximum value of inhaled air flow in a maximum forced inhalation process.

The maximal expiratory pressure indicates a maximum value of oral pressure capable of being generated in the maximum forced exhalation process.

The maximal inspiratory pressure indicates a maximum value of oral pressure capable of being generated in the maximum forced inhalation process.

The maximal voluntary ventilation indicates maximum ventilation in 1 minute.

The maximal mid-expiratory flow indicates a mean expiratory flow rate when 25% to 75% of the vital capacity is exhaled forcibly.

According to the second aspect, in a possible implementation, the first vital capacity standard data is related to a first gender of a first user and a first height of the first user, and the first standard curve includes a first curve, a second curve, and a third curve.

The first curve includes a curve of a change in vital capacity data of a user group of the first gender at the first height with an age. The second curve includes a curve of a change in vital capacity data of the user group of the first gender at a second height with the age.

The third curve includes a curve of a change in vital capacity data of the user group of the first gender at a third height with the age. The second height is greater than the first height, and the third height is greater than the first height.

According to the second aspect, in a possible implementation, the second height includes an average minimum height of the user group of the first gender, and the third height is an average maximum height of the user group of the first gender.

According to the second aspect, in a possible implementation, a difference between the first height and the second height is a first threshold, and a difference between the third height and the first height is a second threshold; and the first threshold is the same as or different from the second threshold.

According to the second aspect, in a possible implementation, the electronic device is further configured to: receive a first operation performed by a user; obtain second vital capacity standard data in response to the first operation; and display a second graph, where the second graph includes a second standard curve corresponding to the second vital capacity standard data and a second graphical marker corresponding to the pulmonary function detection data, and the second vital capacity standard data is related to the first gender of the first user. The second standard curve includes a plurality of curves of changes in vital capacity data of the user group of the first gender at different heights with the age.

According to the second aspect, in a possible implementation, the electronic device is further configured to: display a first user interface, where the first user interface further includes first prompt information, the first prompt information includes identifiers of a plurality of users, and the identifiers of the plurality of users include an identifier of the first user; and receive a first selection operation performed by the user on the identifier of the first user in the first user interface; and the electronic device is specifically configured to obtain the first vital capacity standard data after receiving the selection operation performed on the identifier of the first user.

According to the second aspect, in a possible implementation, the electronic device is further configured to store the pulmonary function detection data in a storage area corresponding to the first user. Different users correspond to different storage areas.

According to the second aspect, in a possible implementation, the first user interface further includes a first control, and the electronic device is further configured to: receive a second operation performed by the user on the first control; and send a first message to the wearable device in response to the second operation. The first message indicates the wearable device to collect the audio data and the physiological data.

According to the second aspect, in a possible implementation, the electronic device is further configured to: display a second user interface, where a second control is displayed in the second user interface; receive a third operation performed by the user on the second control; and display a third graph in response to the third operation. The third graph includes pulmonary function detection data in a first time period.

According to the second aspect, in a possible implementation, the second user interface further includes second prompt information, the second prompt information includes the identifiers of the plurality of users, and the identifiers of the plurality of users include the identifier of the first user; and the electronic device is further configured to: receive a second selection operation performed by the user on the identifier of the first user in the second user interface; and obtain pulmonary function detection data of the first user in the first time period based on the identifier of the first user in response to the second selection operation.

According to the second aspect, in a possible implementation, the audio data includes any one or more of the following: a cough sound, a blow sound, and a speaking sound; and the physiological data includes any one or more of the following: a heart rate, heart rate variability, a respiratory rate, and blood oxygen.

According to a third aspect, this application provides an electronic device. The electronic device includes a processor and a memory, the memory is coupled to the processor, the memory is configured to store computer program code, the computer program code includes computer instructions, and the processor invokes the computer instructions, to perform the health data display method provided in any possible implementation of the first aspect.

According to a fourth aspect, this application provides a computer-readable storage medium, configured to store computer instructions. When the computer instructions are run on an electronic device, a server is enabled to perform the health data display method provided in any possible implementation of the first aspect.

According to a fifth aspect, this application provides a computer program product. When the computer program product runs on an electronic device, the electronic device is enabled to perform the health data display method provided in any possible implementation of the first aspect.

For descriptions of beneficial effects in the second aspect to the fifth aspect, refer to descriptions of beneficial effects in the first aspect. Details are not described herein again in this application.

## EP 4 599 761 A1

## BRIEF DESCRIPTION OF DRAWINGS

FIG. 1 is a diagram of an architecture of a system according to an embodiment of this application;

FIG. 2 is a diagram of a hardware structure of a wearable device 100 according to an embodiment of this application;

FIG. 3 is a diagram of a hardware structure of an electronic device 200 according to an embodiment of this application;

FIG. 4A to FIG. 4C are example diagrams in which an electronic device 200 receives a user operation to enable a pulmonary function detection function;

FIG. 4D to FIG. 4F are diagrams in which a wearable device 100 starts to collect audio data and physiological data;

FIG. 4G to FIG. 4K are diagrams in which an electronic device 200 notifies a user to collect audio data and physiological data;

FIG. 4L to FIG. 4N are diagrams in which an electronic device 200 compares pulmonary function detection data and pulmonary function standard data for display;

FIG. 4O to FIG. 4R are diagrams in which an electronic device 200 detects pulmonary functions of different users;

FIG. 4S to FIG. 4V are diagrams in which an electronic device 200 displays pulmonary function detection data in first duration; and

FIG. 5 is a schematic flowchart of a health data display method according to this application.

## DESCRIPTION OF EMBODIMENTS

The following clearly describes the technical solutions in embodiments of this application in detail with reference to the accompanying drawings. In descriptions of embodiments of this application, unless otherwise specified, "/" indicates "or". For example, A/B may indicate A or B. The term "and/or" in this specification merely describes an association relationship for describing associated objects, and indicates that three relationships may exist. For example, A and/or B may indicate the following three cases: Only A exists, both A and B exist, and only B exists. In addition, in the descriptions of embodiments of this application, "a plurality of" means two or more.

The terms "first" and "second" mentioned below are merely intended for a purpose of description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form that can be accepted by the user. The user interface is source code written in a specific computer language such as Java or an extensible markup language (extensible markup language, XML). Interface source code is parsed and rendered on an electronic device, and is finally presented as content that can be recognized by the user. The user interface is usually represented in a form of a graphical user interface (graphic user interface, GUI), and is a user interface that is related to a computer operation and that is displayed in a graphic manner. The user interface may be visual interface element such as a text, an icon, a button, a menu, a tab, a text box, a dialog box, a status bar, a navigation bar, or a widget displayed on a display of the electronic device.

An embodiment of this application provides a display method. The method may be applied to the physical health detection data display field. Health detection data includes but is not limited to: blood pressure detection data, heart rate detection data, blood oxygen detection data, lung infection detection data, altitude sickness detection data, liver fat detection data, body temperature detection data, pulmonary function detection data, and the like.

After an electronic device obtains the health detection data, the electronic device may further obtain health standard data, and compare the health detection data and the health standard data in a form of a graph for display to a user to view, so that the user can intuitively view, by using an icon, whether health detection data of the user falls within a normal range.

The health standard data may be pre-stored in the device by the electronic device, or the health standard data may be obtained by the electronic device from a server or another device in real time. This is not limited in this application.

For example, the health detection data may be pulmonary function detection data.

When a pulmonary function is abnormal, a lung may have a series of changes. For example, an airway such as a trachea/bronchus secretes mucus, a pulmonary alveolus is ruptured, or elasticity is lost. Consequently, a ventilation function and a gas exchange function of the lung are affected, and a human body has respiratory symptoms such as coughing, dyspnea, decreased blood oxygen, and an accelerated heart rate. Therefore, the pulmonary function may be monitored based on a change in audio output by the user and a change in a physiological parameter.

Specifically, the electronic device may collect audio data and/or physiological data, and obtain the pulmonary function detection data based on the audio data and/or the physiological data. The pulmonary function detection data includes but is not limited to one or more of the following: a vital capacity, a forced expiratory volume in 1 second/forced vital capacity ratio, a forced expiratory volume in 1 second, peak expiratory flow, peak inspiratory flow, maximal expiratory pressure, maximal inspiratory pressure, maximal mid-expiratory flow, and maximal mid-expiratory flow. The pulmonary function detection data may reflect whether the pulmonary function of the monitored user is normal or abnormal.

The vital capacity may indicate a total amount of air that can be exhaled in effective duration when the user inhales air forcibly and deeply and then exhales air at a maximum force and at a maximum speed.

The forced expiratory volume in 1 second may indicate a total amount of air exhaled in a first second when forced air exhalation is performed after maximum deep air inhalation.

The forced expiratory volume in 1 second/forced vital capacity ratio may indicate a percentage of an amount of air exhaled in the first second to a total amount of air exhaled in the effective duration when the user inhales air forcibly and deeply and then exhales air at a maximum force and at a maximum speed.

The peak expiratory flow may indicate an instantaneous maximum value of exhaled air flow in a maximum forced exhalation process.

The peak inspiratory flow may indicate an instantaneous maximum value of inhaled air flow in a maximum forced inhalation process.

The maximal expiratory pressure may indicate a maximum value of oral pressure capable of being generated in the maximum forced exhalation process.

The maximal inspiratory pressure may indicate a maximum value of oral pressure capable of being generated in the maximum forced inhalation process.

The maximal voluntary ventilation may indicate maximum ventilation in 1 minute.

The maximal mid-expiratory flow may indicate a mean expiratory flow rate when 25% to 75% of the vital capacity is exhaled forcibly.

In a possible implementation, the electronic device may obtain the pulmonary function detection data only based on the audio data.

In another possible implementation, the electronic device may alternatively obtain the pulmonary function detection data only based on the physiological data.

In another possible implementation, the electronic device may alternatively obtain the pulmonary function detection data with reference to the audio data and the physiological data. This is not limited in this application.

The audio data includes but is not limited to a cough sound, a blow sound, a normal speaking voice (or referred to as a speaking sound), or the like. The electronic device may detect the audio data by using a microphone.

The physiological data includes but is not limited to one or more of the following: a heart rate, heart rate variability, a respiratory rate, blood oxygen, and the like.

In a possible implementation, the electronic device may detect the respiratory rate of the user by using a motion sensor. The motion sensor may include but is not limited to one or more inertia sensors, one or more acceleration sensors, and the like.

In a possible implementation, the electronic device may detect data such as the heart rate, the heart rate variability, and the blood oxygen of the user by using a photoelectric sensor. Specifically, the photoelectric sensor is configured to monitor a cardiovascular vital sign. The photoelectric sensor includes at least one pair of light-emitting diodes and a photoelectric detector. The light-emitting diode serves as a light source to irradiate skin, and the photoelectric detector detects remaining transmitted or reflected light after light is absorbed by blood and tissue in a penetration process, and converts the remaining transmitted or reflected light into an electrical signal, to obtain a photoplethysmography (photoplethysmography, PPG) signal. Because intensity of the transmitted light or the reflected light changes with arterial pulsation, the PPG signal also follows the arterial pulsation. That is, a heartbeat of the user fluctuates rhythmically. The data such as the heart rate, the heart rate variability, and the blood oxygen of the user may be calculated based on the PPG signal.

In another possible implementation, the electronic device may also detect the data such as the heart rate, the heart rate variability, and a respiratory rate of the user by using an electrocardiogram (electrocardiogram, ECG) sensor.

It should be noted that the electronic device may alternatively obtain the audio data and the physiological data of the user in another manner. This is not limited in this application.

After the electronic device obtains the pulmonary function detection data, the electronic device may further obtain pulmonary function standard data, and compare the pulmonary function detection data and the pulmonary function standard data in a form of a graph for display to the user to view, so that the user can intuitively view, by using an icon, whether the pulmonary function detection data of the user falls within a normal range.

In the following embodiments of this application, a display method provided in this application is described in detail by using examples of how an electronic device collects pulmonary function detection data and how the electronic device displays the pulmonary function detection data. This application is also applicable to other health detection data.

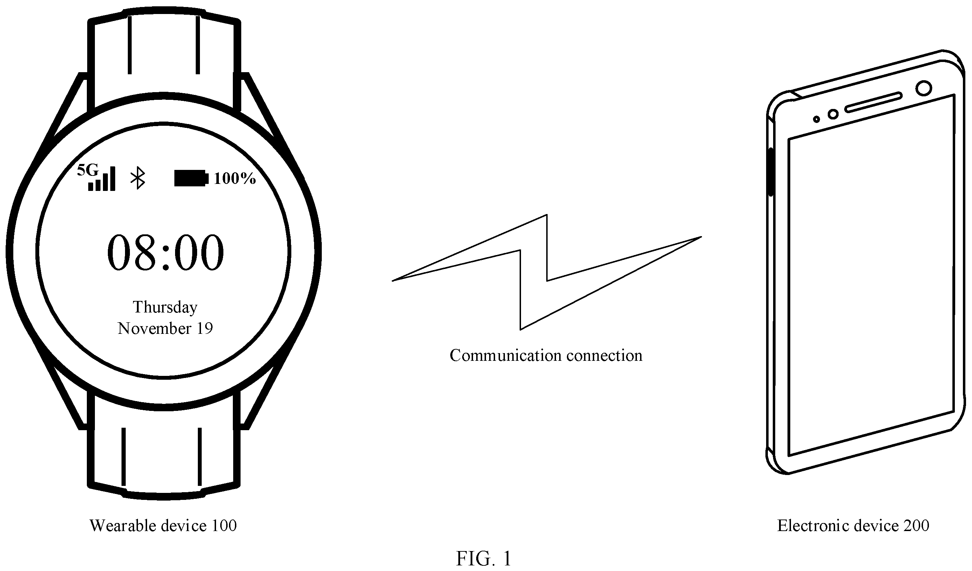

FIG. 1 is a diagram of an architecture of a system according to this application.

As shown in FIG. 1, the system may include a wearable device 100 and an electronic device 200. The wearable device 100 may establish a communication connection to the electronic device 200.

The wearable device 100 may include but is not limited to a device such as a watch or a band.

The electronic device 200 may include but is not limited to devices such as a mobile phone, a tablet computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device (a smartwatch or a smart band), a vehicle-mounted device, a smart home device, and/or a smart city device.

The following embodiments of this application are described by using an example in which the wearable device 100 is a watch and the electronic device 200 is a mobile phone.

The wearable device 100 and the electronic device 200 log in to a same account, and the wearable device 100 may communicate with the electronic device 200 by using a server. Alternatively, the wearable device 100 may establish a Bluetooth connection to the electronic device 200, and the wearable device 100 and the electronic device 200 may communicate with each other through the Bluetooth connection. The wearable device 100 may further communicate with the electronic device 200 in another manner. This is not limited in this application.

The wearable device 100 may collect audio data and physiological data of a user, and send the audio data and the physiological data of the user to the electronic device 200. The electronic device 200 may obtain pulmonary function detection data based on the audio data and the physiological data of the user. The electronic device 200 may further obtain pulmonary function standard data, and display the pulmonary function detection data and the pulmonary function standard data in a user interface of the electronic device 200 in a graph. The user may intuitively view whether the pulmonary function detection data of the user falls within a normal range.

In another embodiment, the wearable device 100 may collect audio data and physiological data of a user, and obtain pulmonary function detection data based on the audio data and the physiological data of the user. The wearable device 100 then sends the pulmonary function detection data to the electronic device 200, so that the electronic device 200 can display the pulmonary function detection data.

In another embodiment, the wearable device 100 may collect audio data and physiological data of a user, and obtain pulmonary function detection data based on the audio data and the physiological data of the user. After the wearable device 100 obtains the pulmonary function detection data, the wearable device 100 may obtain pulmonary function standard data, and display the pulmonary function detection data and the pulmonary function standard data in a user interface of the wearable device 100 in a graph.

In another embodiment, the electronic device 200 may alternatively collect audio data and physiological data of a user, and obtain pulmonary function detection data based on the audio data and the physiological data of the user. The electronic device 200 may further obtain pulmonary function standard data, and display the pulmonary function detection data and the pulmonary function standard data in a user interface of the electronic device 200 in a graph.

In the foregoing embodiments, the electronic device 200 and the wearable device 100 may alternatively jointly collect the pulmonary function detection data. For example, the electronic device 200 collects the audio data, and the wearable device 100 collects the physiological data.

In the foregoing embodiments, the electronic device 200 and the wearable device 100 may alternatively separately collect the audio data and the physiological data. This is not limited in this application either.

This application is described by using an example in which the wearable device 100 collects the audio data and the physiological data, and the electronic device 200 obtains the pulmonary function detection data based on the audio data and the physiological data of the user.

The following describes a hardware structure of a wearable device in embodiments of this application.

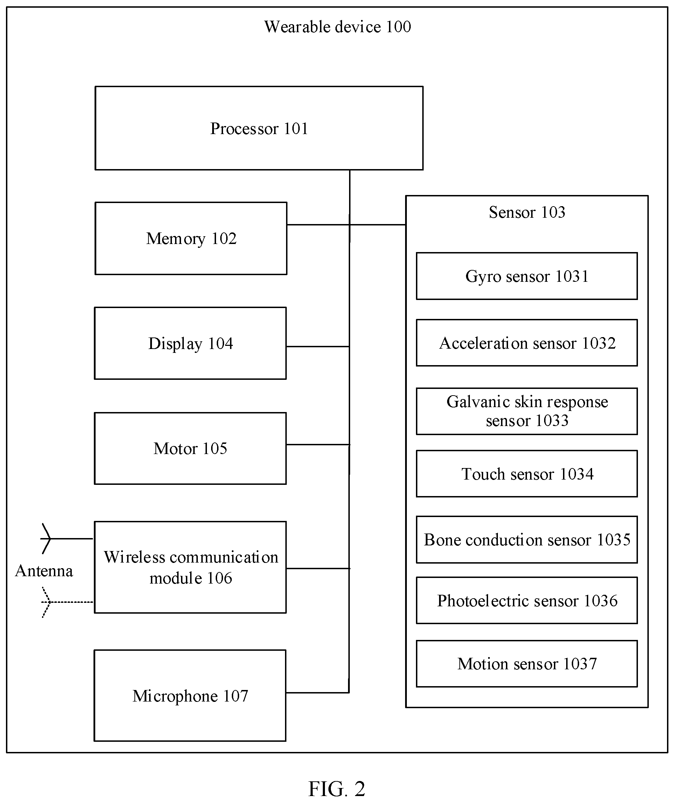

FIG. 2 is a diagram of a hardware structure of a wearable device 100.

The wearable device 100 may be a wearable device such as a smart band or a smartwatch. A specific type of the wearable device 100 is not limited in this embodiment of this application.

The wearable device 100 may include a processor 101, a memory 102, a sensor 103, a display 104, a motor 105, a wireless communication module 106, a microphone 107, and the like.

The processor 101 may include one or more processing units. For example, the processor 101 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on an instruction operation code and a time sequence signal, to complete control of instruction reading and instruction execution.

A memory may be further disposed in the processor 101, and is configured to store instructions and data. In some embodiments, the memory in the processor 101 is a cache memory. The memory may store instructions or data that is just used or repeatedly used by the processor 101. If the processor 101 needs to use the instructions or the data again, the processor may directly invoke the instructions or the data from the memory. This avoids repeated access, reduces waiting time of the processor 101, and improves system efficiency.

The memory 102 may include one or more random access memories (random access memory, RAM) and one or more nonvolatile memories (nonvolatile memory, NVM).

The random access memory may be directly read and written by the processor 101, and may be configured to store an executable program (for example, a machine instruction) of an operating system or another running program, and may be further configured to store data of a user and data of an application.

The nonvolatile memory may store an executable program, data of a user and an application, and the like, and may be loaded into the random access memory in advance, so that the processor 101 directly performs reading and writing.

The sensor 103 may include a plurality of sensors such as a gyro sensor 1031, an acceleration sensor 1032, a galvanic skin response sensor 1033, a touch sensor 1034, a bone conduction sensor 1035, a photoelectric sensor 1036, and a motion sensor 1037.

The gyro sensor 1031 may be configured to determine a moving posture of the wearable device 100. In some embodiments, angular velocities of the wearable device 100 around three axes (namely, axes x, y, and z) may be determined through the gyro sensor 1031.

The acceleration sensor 1032 detects acceleration values of the wearable device 100 in various directions (usually on three axes). A magnitude and a direction of gravity may be detected when the wearable device 100 is still. The acceleration sensor 1032 may be further configured to identify a posture of an electronic device, and is used in an application such as switching between a landscape mode and a portrait mode or a pedometer.

The galvanic skin response sensor 1033 may be configured to measure electrodermal activity data of the user, and the data reflects a change in pressure and emotion of the user.

The touch sensor 1034 is also referred to as a "touch component". The touch sensor 1034 may be disposed on the display 104, and the touch sensor 1034 and the display 104 constitute a touchscreen, which is also referred to as a "touchscreen". The touch sensor 1034 is configured to detect a touch operation performed on or near the touch sensor. The touch sensor may transfer the detected touch operation to the application processor to determine a type of a touch event. A visual output related to the touch operation may be provided on the display 104. In some other embodiments, the touch sensor 1034 may alternatively be disposed on a surface of the wearable device 100, and is located at a location different from that of the display 104.

In this embodiment of this application, the wearable device 100 may detect, by using the touch sensor 1034, a touch operation performed by the user on the display 104.

The bone conduction sensor 1035 may obtain a vibration signal. In some embodiments, the bone conduction sensor 1035 may obtain a vibration signal of a vibration bone of a human vocal-cord part. The bone conduction sensor 1035 may also contact a pulse of a human body, to receive a blood pressure beating signal. In some embodiments, the bone conduction sensor 1035 may also be disposed in the headset, to obtain a bone conduction headset. The application processor may parse heart rate information based on the blood pressure beating signal obtained by the bone conduction sensor 1035, to implement a heart rate detection function.

The photoelectric sensor 1036 is configured to monitor a cardiovascular vital sign. The photoelectric sensor 1036 includes at least one pair of light-emitting diodes and a photoelectric detector. The light-emitting diode serves as a light source to irradiate skin, and the photoelectric detector detects remaining transmitted or reflected light after light is absorbed by blood and tissue in a penetration process, and converts the remaining transmitted or reflected light into an electrical signal, to obtain a PPG signal. Because intensity of the transmitted light or the reflected light changes with arterial pulsation, the PPG signal also follows the arterial pulsation. That is, a heartbeat of the user fluctuates rhythmically. Parameters such as a heart rate, heart rate variability, blood oxygen, and blood pressure of the user may be calculated based on the PPG signal.

The motion sensor 1037 is configured to: collect motion data, for example, acceleration data, and detects, based on the motion data, whether the wearable device 100 is in a moving state or a still state. When detecting heart rate data, the wearable device 100 determines, based on the motion data collected by the motion sensor 1037, that the wearable device 100 is in the moving state. In this case, the wearable device 100 may notify that current pulmonary function detection is invalid, and notify the user to detect a pulmonary function when the user is in the still state, to avoid impact of motion on a result of the pulmonary function detection.

It may be understood that the sensor 103 may include more or fewer sensors, for example, an ECG sensor. This is not limited in this embodiment of this application.

The display 104 may be configured to display an image, a video, and the like.

The motor 105 may generate a vibration prompt. The motor 105 may be configured to provide an incoming call vibration prompt and a touch vibration feedback. For example, touch operations performed on different applications (for example, photographing or audio playing) may correspond to different vibration feedback effects.

The wireless communication module 106 may provide a wireless communication solution that is applied to the wearable device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 106 may be one or more components integrating at least one communication processing module. The wireless communication module 106 receives an electromagnetic wave through the antenna, performs demodulation and filtering processing on an electromagnetic wave signal, and sends a processed signal to the processor 101. The wireless communication module 106 may further receive a to-be-sent signal from the processor 101, perform frequency modulation and amplification on the signal, and convert the signal into an electromagnetic wave for radiation through the antenna.

In some embodiments, the antenna and the wireless communication module 106 in the wearable device 100 are coupled, so that the wearable device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (beidou navigation satellite system, BDS), a quasi-zenith satellite system (quasi-zenith satellite system, QZSS), and/or a satellite based augmentation system (satellite based augmentation system, SBAS).

The microphone 107, also referred to as a "mike" or a "mic", is configured to convert a sound signal into an electrical signal. When making a call or sending a voice message, the user may make a sound near the microphone 107 through the mouth of the user, to input a sound signal to the microphone 107. At least one microphone 107 may be disposed in the wearable device 100. In some other embodiments, two microphones 107 may be disposed in the wearable device 100, to collect a sound signal and further implement a noise reduction function. In some other embodiments, three, four, or more microphones 107 may alternatively be disposed in the wearable device 100, to collect a sound signal, reduce noise, identify a sound source, implement a directional recording function, and the like. In this embodiment of this application, the microphone 107 may be configured to collect audio data of the user, for example, a cough sound, a blow sound, or a voice.

It can be understood that the structure shown in this embodiment of this application does not constitute a specific limitation on the wearable device 100. In some other embodiments of this application, the wearable device 100 may include more or fewer components than those shown in the figure, some components may be combined, or some components may be divided, or different component arrangements may be used. The components shown in the figure may be implemented as hardware, software, or a combination of software and hardware.

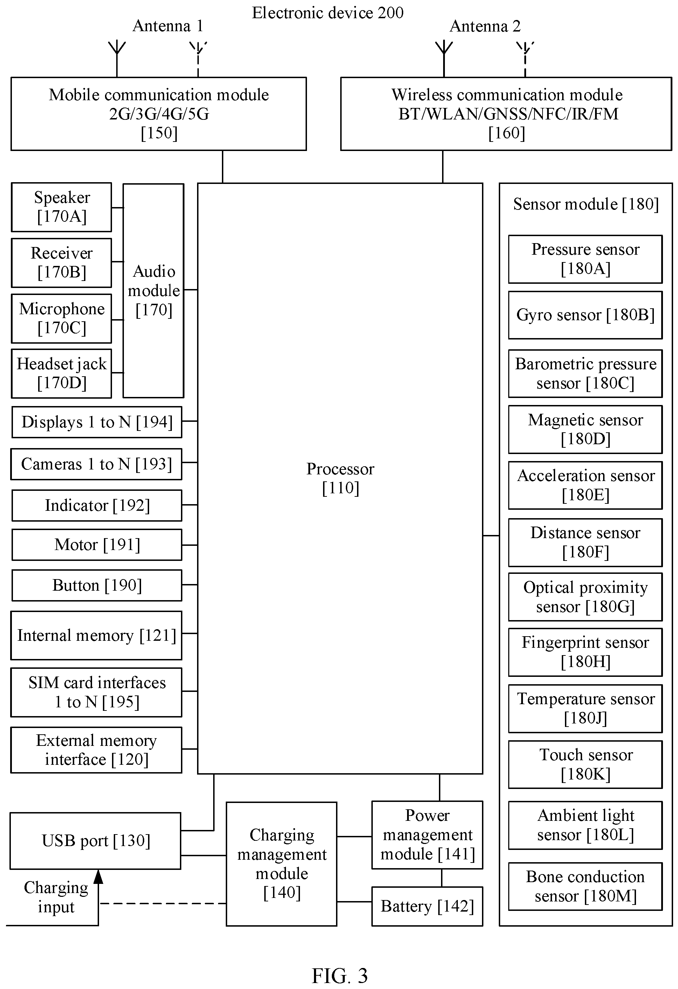

FIG. 3 is a diagram of a hardware structure of an electronic device 200 according to an embodiment of this application.

The electronic device 200 may include a processor 110, an external memory interface 120, an internal memory 121, a universal serial bus (universal serial bus, USB) port 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that a structure shown in this embodiment of the present invention does not constitute a specific limitation on the electronic device 200. In some other embodiments of this application, the electronic device 200 may include more or fewer components than those shown in the figure, some components may be combined, or some components may be divided, or different component arrangements may be used. The components shown in the figure may be implemented as hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache memory. The memory may store instructions or data that is just used or repeatedly used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/-transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) port, and/or the like.

The USB port 130 is an interface that conforms to a USB standard specification, and may be specifically a mini USB port, a micro USB port, a USB Type C port, or the like.

It may be understood that a port connection relationship between the modules that is shown in this embodiment of the present invention is merely an example for description, and does not constitute a limitation on the structure of the electronic device 200. In some other embodiments of this application, different interface connection manners in the foregoing embodiments or a combination of a plurality of interface connection manners may alternatively be used for the electronic device 200.

The charging management module 140 is configured to receive a charging input from a charger. The charging management module 140 may further supply power to the electronic device by using the power management module 141 while charging the battery 142.

The power management module 141 is configured to be connected to the battery 142, the charging management module 140, and the processor 110. In some other embodiments, the power management module 141 may alternatively be disposed in the processor 110. In some other embodiments, the power management module 141 and the charging management module 140 may alternatively be disposed in a same device.

A wireless communication function of the electronic device 200 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 200 may be configured to cover one or more communication frequency bands. Different antennas may be further multiplexed, to improve antenna utilization. For example, the antenna 1 may be multiplexed as a diversity antenna in a wireless local area network. In some other embodiments, the antenna may be used in combination with a tuning switch.

The mobile communication module 150 may provide a wireless communication solution that is applied to the electronic device 200 and that includes a 2G/3G/4G/5G or the like.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal.

The wireless communication module 160 may provide a wireless communication solution that is applied to the electronic device 200 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, the antenna 1 and the mobile communication module 150 in the electronic device 200 are coupled, and the antenna 2 and the wireless communication module 160 in the electronic device 200 are coupled, so that the electronic device 200 can communicate with a network and another device by using a wireless communication technology.

The electronic device 200 may implement a display function through the GPU, the display 194, the application processor, and the like. The GPU is a microprocessor for image processing, and is connected to the display 194 and the application processor. The GPU is configured to: perform mathematical and geometric computation, and render an image.

The processor 110 may include one or more GPUs, and execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flex light-emitting diode, FLED), a mini-LED, a micro-LED, a micro-OLED, a quantum dot light emitting diode (quantum dot light emitting diode, QLED), or the like. In some embodiments, the electronic device 200 may include one or N displays 194. N is a positive integer greater than 1.

The electronic device 200 can implement a photographing function through the ISP, the camera 193, the video codec, the GPU, the display 194, the application processor, and the like.

The ISP is configured to process data fed back by the camera 193. For example, during photographing, a shutter is pressed, and light is transferred to a photosensitive element of the camera through a lens. An optical signal is converted into an electrical signal, and the photosensitive element of the camera transfers the electrical signal to the ISP for processing, to convert the electrical signal into a visible image. The ISP may further perform algorithm optimization on noise, and brightness of the image. The ISP may further optimize parameters such as exposure and a color temperature of a photographing scenario. In some embodiments, the ISP may be disposed in the camera 193.

The camera 193 is configured to capture a static image or a video. An optical image of an object is generated through the lens, and is projected onto a photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format such as RGB or YUV. In some embodiments, the electronic device 200 may include one or N cameras 193. N is a positive integer greater than 1.

The digital signal processor is configured to process a digital signal, and may process another digital signal in addition to the digital image signal. For example, when the electronic device 200 selects a frequency, the digital signal processor is configured to perform Fourier transform on frequency energy.

The video codec is configured to compress or decompress a digital video. The electronic device 200 may support one or more video codecs. Therefore, the electronic device 200 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor. By referring to a structure of a biological neural network, for example, a transfer mode between human brain neurons, the NPU quickly processes input information, and may further continuously perform self-learning. Applications such as intelligent cognition of the electronic device 200 may be implemented by using the NPU, for example, image recognition, facial recognition, speech recognition, and text understanding.

The internal memory 121 may include one or more random access memories (random access memory, RAM) and one or more nonvolatile memories (nonvolatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, such as a 5th generation DDR SDRAM generally referred to as DDR5 SDRAM), or the like. The non-volatile memory may include a magnetic disk storage device and a flash memory (flash memory).

The flash memory may include a NOR flash, a NAND flash, a 3D NAND flash, and the like based on an operating principle; may include a single-level cell (single-level cell, SLC), a multi-level cell (multi-level cell, MLC), a triple-level cell (triple-level cell, TLC), a quad-level cell (quad-level cell, QLC), and the like based on a quantity of electric potential levels of a cell; and may include a universal flash storage (English: universal flash storage, UFS), an embedded multimedia card (embedded multimedia Card, eMMC), and the like based on a storage specification.

The random access memory may be directly read and written by the processor 110, and may be configured to store an executable program (for example, a machine instruction) of an operating system or another running program, and may be further configured to store data of a user and data of an application.

The nonvolatile memory may store an executable program, data of a user and an application, and the like, and may be loaded into the random access memory in advance, so that the processor 110 directly performs reading and writing.

The external memory interface 120 may be configured to be connected to an external nonvolatile memory, to expand a storage capacity of the electronic device 200. The external nonvolatile memory communicates with the processor 110 through the external memory interface 120, to implement a data storage function. For example, a file such as music or a video is stored in the external nonvolatile memory.

The electronic device 200 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "horn", is configured to convert an audio electrical signal into a sound signal. The electronic device 200 may be used to listen to music or answer a call in a hands-free mode over the speaker 170A.

The receiver 170B, also referred to as an "earpiece", is configured to convert an audio electrical signal into a sound signal. When a call is answered or speech information is received through the electronic device 200, the receiver 170B may be put close to a human ear to listen to a voice.

The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 200. In some other embodiments, two microphones 170C may be disposed in the electronic device 200, to collect a sound signal and implement a noise reduction function. In some other embodiments, three, four, or more microphones 170C may alternatively be disposed in the electronic device 200, to collect a sound signal, reduce noise, identify a sound source, implement a directional recording function, and the like.

The headset jack 170D is configured to be connected to a wired headset. The headset jack 170D may be the USB port 130, or may be a 3.5 mm open mobile terminal platform (open mobile terminal platform, OMTP) standard interface, or a cellular telecommunications industry association of the USA (cellular telecommunications industry association of the USA, CTIA) standard interface.

The pressure sensor 180A is configured to sense a pressure signal, and can convert the pressure signal into an electrical signal.

The gyro sensor 180B may be configured to determine a moving posture of the electronic device 200.

The barometric pressure sensor 180C is configured to measure barometric pressure. In some embodiments, the electronic device 200 calculates an altitude through the barometric pressure measured by the barometric pressure sensor 180C, to assist in positioning and navigation.

The magnetic sensor 180D includes a Hall sensor. The electronic device 200 may detect opening and closing of a flip cover by using the magnetic sensor 180D.

The acceleration sensor 180E may detect accelerations of the electronic device 200 in various directions (usually on three axes). A magnitude and a direction of gravity may be detected when the electronic device 200 is still. The acceleration sensor 180E may be further configured to identify a posture of the electronic device, and is used in an application such as switching between a landscape mode and a portrait mode or a pedometer.

The distance sensor 180F is configured to measure a distance. The electronic device 200 may measure the distance in an infrared manner or a laser manner.

The optical proximity sensor 180G may include, for example, a light-emitting diode (LED) and an optical detector, for example, a photodiode.

The ambient light sensor 180L is configured to sense ambient light brightness. The electronic device 200 may adaptively adjust brightness of the display 194 based on the sensed ambient light brightness.

The fingerprint sensor 180H is configured to collect a fingerprint.

The temperature sensor 180J is configured to detect a temperature.

The touch sensor 180K is also referred to as a "touch component".

The bone conduction sensor 180M may obtain a vibration signal.

The button 190 includes a power button, a volume button, and the like. The button 190 may be a mechanical button, or may be a touch button. The electronic device 200 may receive a key input, and generate a key signal input related to a user setting and function control of the electronic device 200.

The motor 191 may generate a vibration prompt.

The indicator 192 may be an indicator light, and may be configured to indicate a charging status and a power change, or may be configured to indicate a message, a missed call, a notification, and the like.

The SIM card interface 195 is configured to be connected to an SIM card.

It may be understood that a structure shown in this embodiment of the present invention does not constitute a specific limitation on the electronic device 200. In some other embodiments of this application, the electronic device 200 may include more or fewer components than those shown in the figure, some components may be combined, or some components may be divided, or different component arrangements may be used. The components shown in the figure may be implemented as hardware, software, or a combination of software and hardware.

When a user needs to detect a pulmonary function, an electronic device 200 may receive a user operation to enable a pulmonary function detection function. Therefore, the electronic device 200 may send a message to a wearable device 100 after the pulmonary function detection function is enabled. In response to the message, the wearable device 100 may enable a microphone and a sensor, and start to collect audio data and physiological data. Therefore, when the pulmonary function does not need to be detected, the wearable device 100 may disable the microphone and the sensor, to reduce power consumption of the wearable device 100.

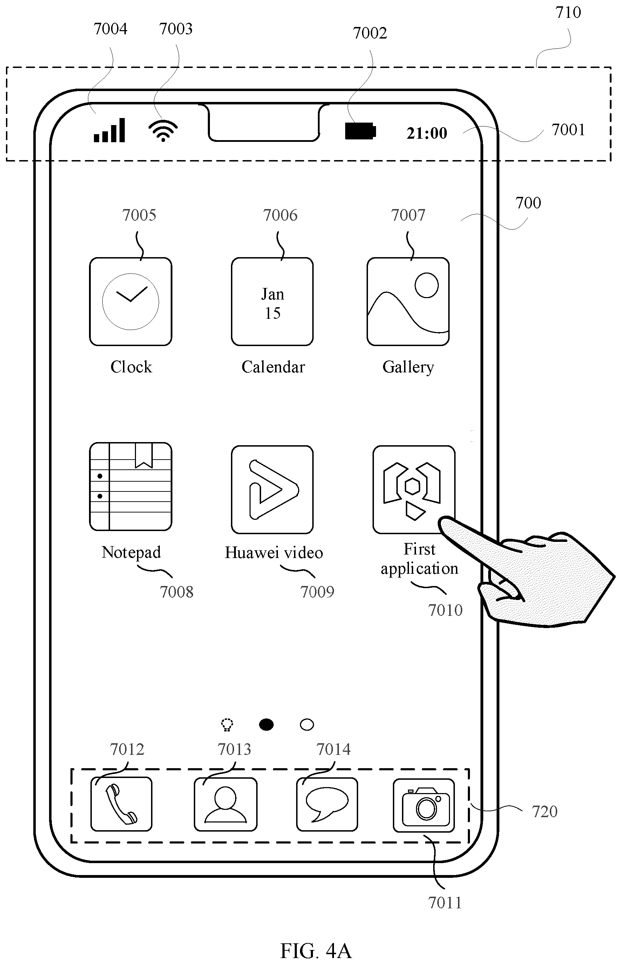

FIG. 4A to FIG. 4C are example diagrams in which an electronic device 200 receives a user operation to enable a pulmonary function detection function.

As shown in FIG. 4A, the electronic device 200 displays a home screen user interface 700. The user interface 700 may include a status bar 710, a tray 720 having a frequently used application icon, and another application icon. The status bar 710 may include a time indicator 7001, a battery status indicator 7002, one or more signal strength indicators 7003 of a wireless fidelity (wireless fidelity, Wi-Fi) signal, and one or more signal strength indicators 7004 of a mobile communication signal (which may also be referred to as a cellular signal). The tray 720 having a frequently used application icon may display a Camera icon 7011, a Phone icon 7012, a Contacts icon 7013, and a Messaging icon 7014. Other application icons may be, for example, an icon 7005 of Clock, an icon 7006 of Calendar, an icon 7007 of Gallery, an icon 7008 of Notepad, an icon 7009 of Huawei video, and an icon 7010 of First application. An icon of any application may be used to respond to a user operation (for example, a single-tap operation), so that the electronic device 200 starts the application corresponding to the icon. The icon 7010 of First application may be used to start First application. The electronic device 200 may detect physical health of the user by using First application.

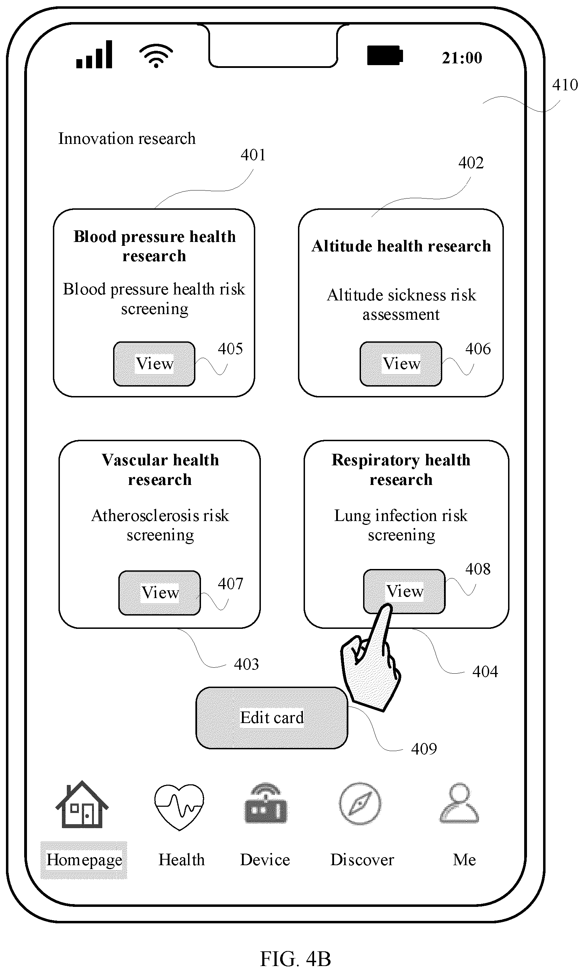

The electronic device 200 may display a user interface 410 shown in FIG. 4B in response to an input operation (for example, a single-tap) performed by the user on the icon 7010 of First application.

The user interface 410 shows one or more options, and the user may select any option to detect health data corresponding to the option. For example, the user interface 410 includes an option 401, an option 402, an option 403, and an option 404.

The user may select a control 405 in the option 401 to trigger the electronic device 200 or the wearable device 100 to detect a blood pressure value of the user.

The user may select a control 406 in the option 402 to trigger the electronic device 200 or the wearable device 100 to detect whether the user has altitude sickness.

The user may select a control 407 in the option 403 to trigger the electronic device 200 or the wearable device 100 to detect whether the user has an atherosclerosis risk.

The user may select a control 408 in the option 404 to trigger the electronic device 200 or the wearable device 100 to detect the pulmonary function of the user, to determine whether the user has lung infection.

It should be noted that only some physical health detection options are shown in the user interface 410.

Optionally, a plurality of physical health detection options shown in the user interface 410 may be frequently used by the user. In this case, the user may place a frequently used physical health detection option in the user interface 410, to facilitate use by the user.

Optionally, the user interface 410 further includes an option 409. The electronic device 200 may receive the user operation, and rearrange one or more physical health detection options displayed in the user interface 410. For example, some physical health detection options are deleted from the user interface 410, and another new physical health detection option is added and displayed in the user interface 410.

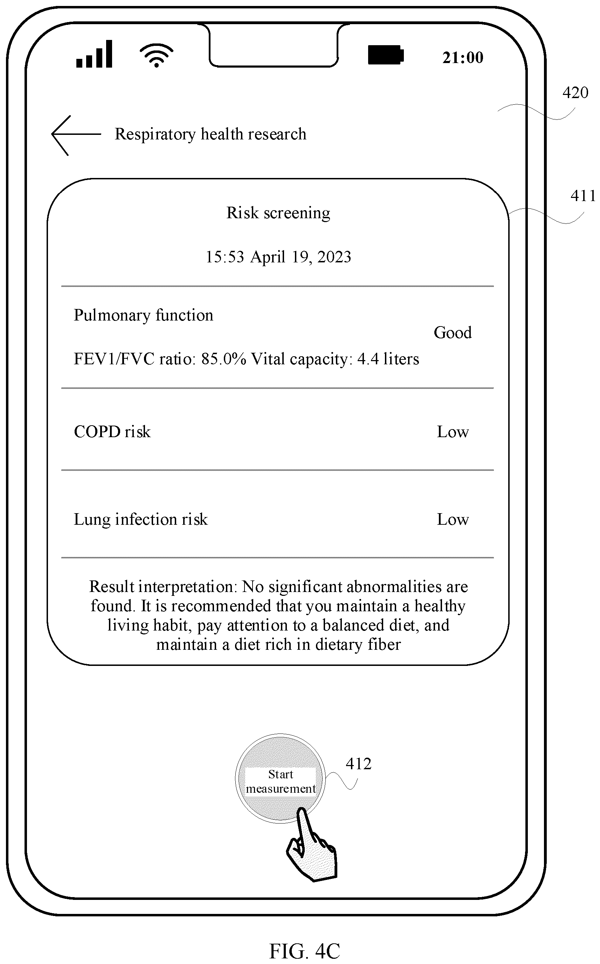

As shown in FIG. 4B, when the user needs to detect the pulmonary function, the electronic device 200 may receive an input operation (for example, a single-tap) performed by the user on the control 408 in the option 404, and in response to the input operation performed by the user, the electronic device 200 may display a user interface 420 shown in FIG. 4C.

Detection information 411 and a control 412 are shown in the user interface 420. The detection information 411 may be pulmonary function detection data of the user during previous detection. Time of previous pulmonary function detection may be 15:53 on April 19, 2023. Previous pulmonary function detection data may include: a forced expiratory volume in 1 second/forced vital capacity ratio is 85%, a vital capacity is 4.4 liters, a pulmonary function is good, a chronic obstructive pulmonary disease risk is a low risk, and a lung infection risk is a low risk. Based on the previous pulmonary function detection data, a given suggestion is "No significant abnormalities are found. It is recommended that you maintain a healthy living habit, pay attention to a balanced diet, and maintain a diet rich in dietary fiber".

In this way, the user may view the previous pulmonary function detection data in the user interface 420.

As shown in FIG. 4C, when the user needs to detect the pulmonary function, the electronic device 200 may receive an input operation (for example, a single-tap) performed by the user on the control 412 in the user interface 420, and in response to the input operation performed by the user, the electronic device 200 and/or the wearable device 100 may start to measure the pulmonary function detection data.

The following embodiments of this application are described by using an example in which the wearable device 100 collects the audio data and the physiological data, and the electronic device 200 displays the pulmonary function detection data.

Optionally, after the electronic device 200 receives the input operation performed by the user on the control 412 in the user interface 420, the electronic device 200 may send a message to the wearable device 100. The message is used to indicate the wearable device 100 to enable the microphone and the sensor and start to collect the audio data and the physiological data.

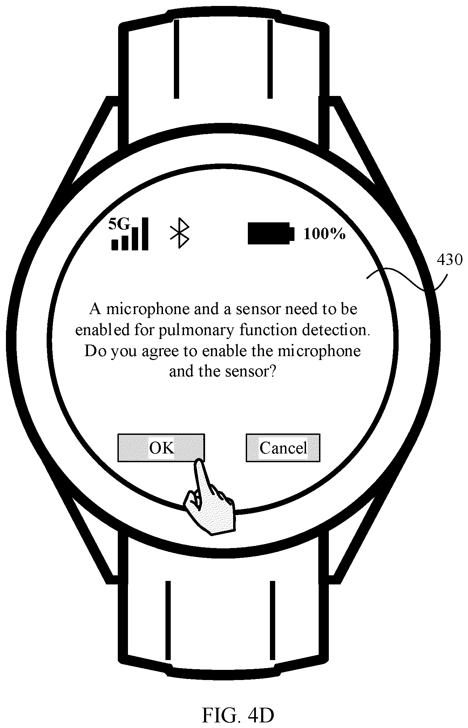

FIG. 4D to FIG. 4F are diagrams in which a wearable device 100 starts to collect audio data and physiological data.

In response to the message sent by the electronic device 200, the wearable device 100 may display a user interface 430 shown in FIG. 4D.

The user interface 430 includes prompt content, and the prompt content is used to notify "A microphone and a sensor need to be enabled for pulmonary function detection. Do you agree to enable the microphone and the sensor?". The sensor may include a motion sensor, a galvanic skin response sensor, and the like.

An "OK" control and a "Cancel" control are also shown in the user interface 430. The wearable device 100 may be configured to receive an input operation (for example, a single-tap) performed by the user on the "OK" control in the user interface 430, and in response to the input operation performed by the user, the wearable device 100 may enable the microphone and the sensor. The wearable device 100 may also be configured to receive an input operation (for example, a single-tap) performed by the user on the "Cancel" control in the user interface 430, and in response to the input operation performed by the user, the wearable device 100 may not enable the microphone and the sensor.

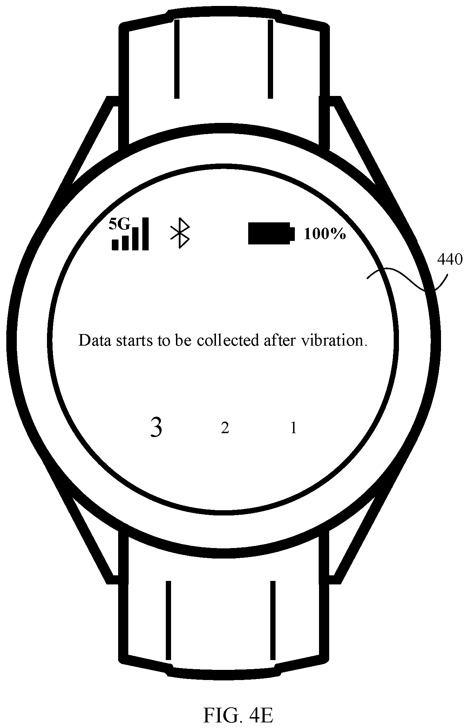

As shown in FIG. 4D, the wearable device 100 may receive the input operation (for example, the single-tap) performed by the user on the "OK" control in the user interface 430, and in response to the input operation performed by the user, the wearable device 100 may enable the microphone and the sensor, and display a user interface 440 shown in FIG. 4E.

Prompt information is displayed in the user interface 440, and the prompt information is used to notify the user of "Data starts to be collected after vibration". For example, the wearable device may vibrate and start to collect data after countdown ends.

Optionally, if the microphone and the sensor in the wearable device 100 are in an enabled state, the wearable device 100 may not display the user interface 430 shown in FIG. 4D, but directly displays the user interface 440 shown in FIG. 4E, to notify the user that the pulmonary function starts to be monitored.

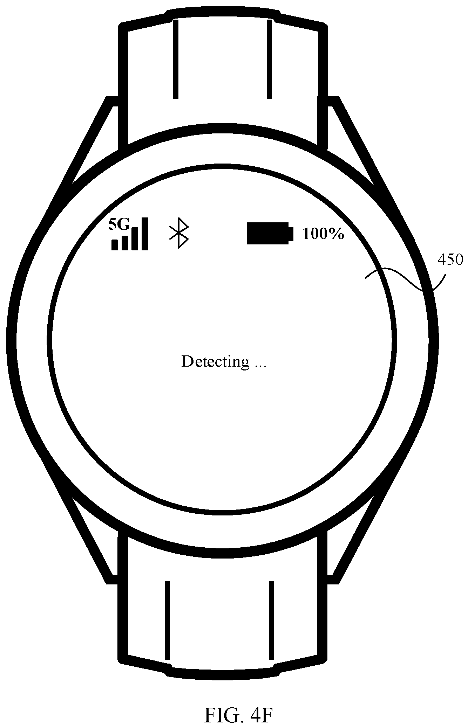

Optionally, in a process in which the wearable device 100 collects the audio data and the physiological data, the wearable device 100 may display a user interface 450 shown in FIG. 4F. Prompt information "Detecting ..." is displayed in the user interface 450, and the prompt information may be used to notify the user that the audio data and the physiological data are currently being collected.

Optionally, in the process in which the wearable device 100 collects the audio data and the physiological data, the wearable device 100 may enable a do-not-disturb mode, to avoid a case in which a new message prompt or an incoming call prompt occurs in the process in which the wearable device 100 collects the audio data and the physiological data, and consequently, data collection by the wearable device 100 is affected.

In another embodiment, the electronic device 200 may alternatively notify the user to enable the microphone and the sensor of the wearable device 100. This is not limited in this application.

Optionally, after the wearable device 100 enables the microphone and the sensor, the wearable device 100 may send a message to the electronic device 200. The message indicates that the wearable device 100 has enabled the microphone and the sensor and has started to collect data. The electronic device 200 may notify the user to collect the audio data and the physiological data by using a correct posture, and notify the user of valid time of collecting the audio data and the physiological data.

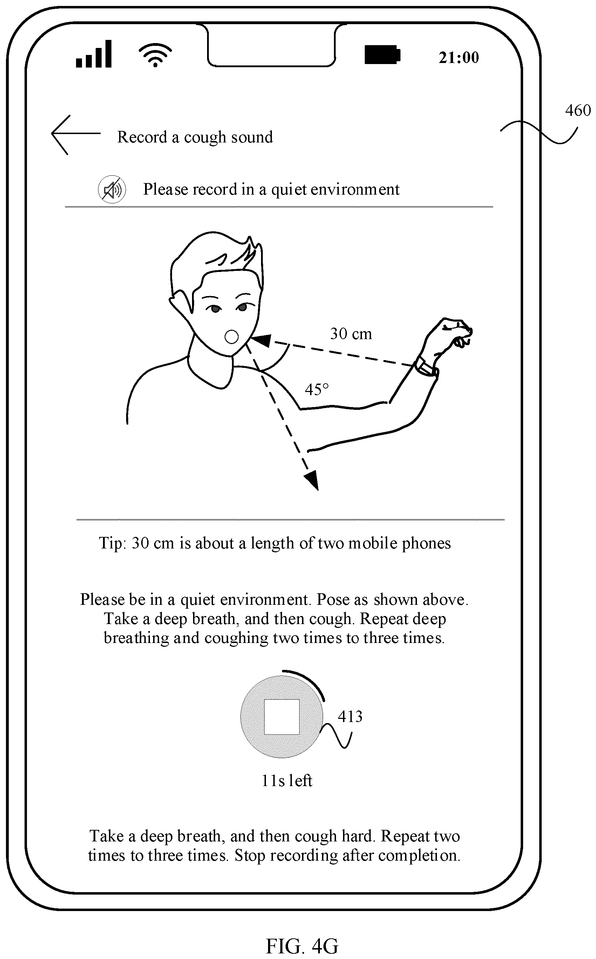

FIG. 4G to FIG. 4K are diagrams in which an electronic device 200 notifies a user to collect audio data and physiological data.

In response to the message sent by the wearable device 100, in a process in which the wearable device 100 collects audio data of the user, the electronic device 200 may display a user interface 460 shown in FIG. 4G. The user interface 460 is configured to notify the user that the wearable device 100 is collecting the audio data, and the user may output audio based on prompt information in the user interface 460 and a correct posture.

For example, a posture of the user may be "A distance between a mouth and a wearable device 100 is 30 centimeters, and an included angle between the mouth and the wearable device 100 is 45 degrees".

The user interface 460 also shows a detection requirement for collecting the audio data. For example, the detection requirement may be "Please be in a quiet environment. Pose as shown above. Take a deep breath, and then cough. Repeat deep breathing and coughing two times to three times".

An icon 413 is also shown in the user interface 460. The icon 413 is configured to display remaining collection duration of the audio data. For example, the remaining collection duration is 11 seconds.

In a possible implementation, if the user has completed collection of the audio data according to the detection requirement before the remaining collection duration reaches 0, the electronic device 200 may receive an input operation (for example, a single-tap) performed by the user on the icon 413, and in response to the input operation performed by the user, the electronic device 200 may stop the countdown.

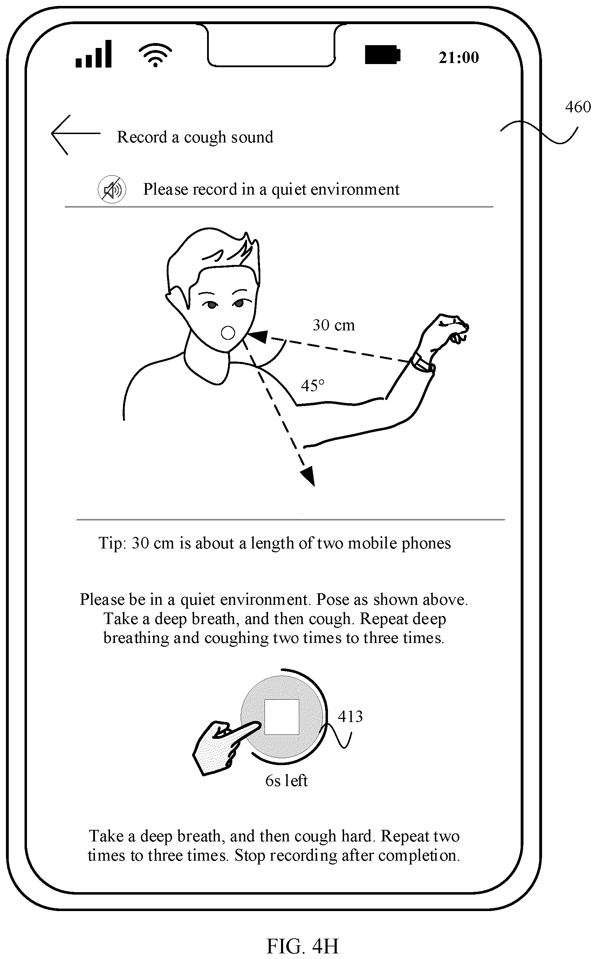

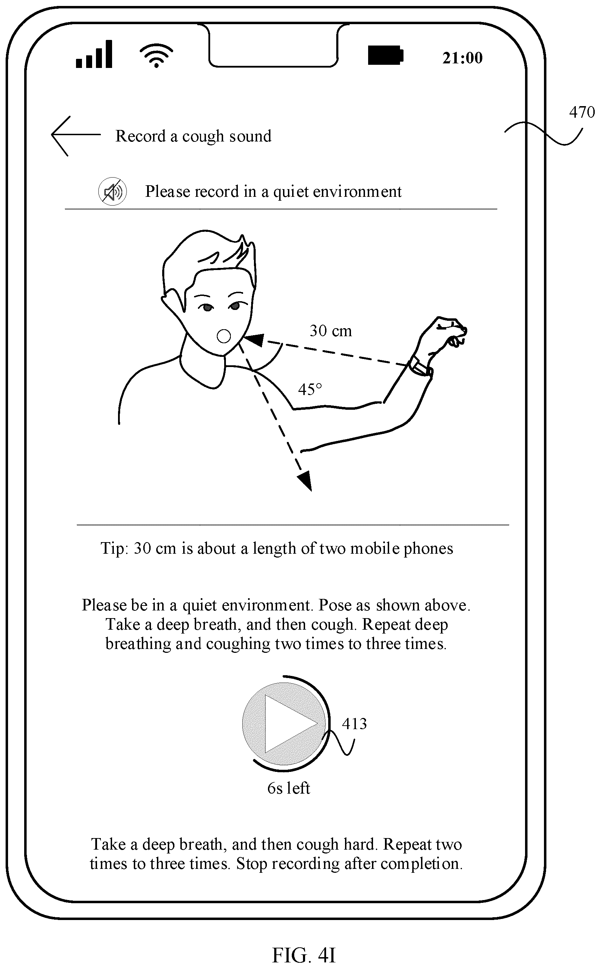

For example, as shown in FIG. 4H, when the remaining collection duration reaches 6 seconds, the electronic device 200 may receive the input operation (for example, the single-tap) performed by the user on the icon 413, and in response to the input operation performed by the user, the electronic device 200 may display a user interface 470 shown in FIG. 4I. In the user interface 470, the electronic device 200 changes a display form of the icon 413, to notify the user that collection of the audio data is stopped.

In some embodiments, after the user actively triggers stopping of collection of the audio data, the electronic device 200 may send a message to the wearable device 100. The message indicates the wearable device 100 to stop collection of the audio data. In a possible implementation, after receiving the message sent by the electronic device 200, the wearable device 100 may disable the microphone, and stop collection of the audio data. In another possible implementation, after receiving the message sent by the electronic device 200, the wearable device 100 may not disable the microphone, but needs to stop collection of the audio data. The wearable device 100 may determine, based on the collected audio data, whether the audio data is valid. When the audio data is valid, the wearable device 100 may disable the microphone, to avoid repeatedly enabling and disabling the microphone.

In another embodiment, if the user has completed collection of the audio data according to the detection requirement before the remaining collection duration reaches 0, after the remaining collection duration reaches 0, the electronic device 200 may send a message to the wearable device 100. The message indicates the wearable device 100 to stop collection of the audio data. In a possible implementation, after receiving the message sent by the electronic device 200, the wearable device 100 may disable the microphone, and stop collection of the audio data. In another possible implementation, after receiving the message sent by the electronic device 200, the wearable device 100 may not disable the microphone, but needs to stop collection of the audio data. The wearable device 100 may determine, based on collected audio data, whether the audio data is valid. When the audio data is valid, the wearable device 100 may disable the microphone, to avoid repeatedly enabling and disabling the microphone.

That the audio data is valid may be that recording duration of the audio data reaches preset duration, and/or a feature of the audio data satisfies a preset feature (for example, a feature of a cough sound, a feature of a speaking sound, or a feature of a blow sound).

After the wearable device 100 stops collection of the audio data, if the wearable device 100 determines that the collected audio data is valid, the wearable device 100 may send a message to the electronic device 200. The message indicates the electronic device 200 to start to notify the user to collect physiological data of the user.

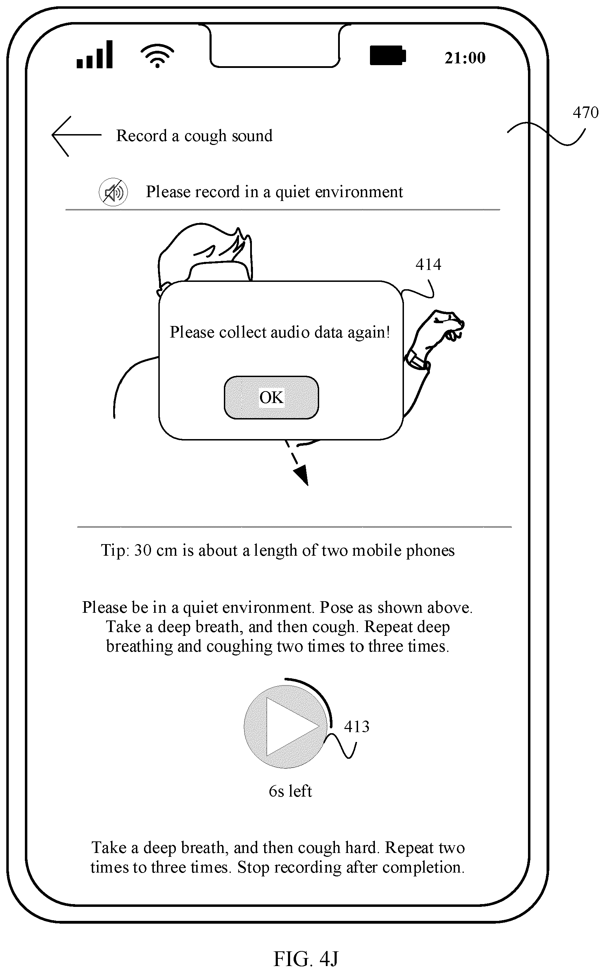

After the wearable device 100 stops collection of the audio data, if the wearable device 100 determines that previously collected audio data is invalid, the wearable device 100 may enable the microphone, and send a message to the electronic device 200. The message indicates the electronic device 200 to notify the user to collect the audio data again. For example, as shown in FIG. 4J, the electronic device 200 may display prompt information 414. The prompt information 414 is used to notify the user to collect the audio data again. After the electronic device 200 receives an input operation (for example, a single-tap operation) performed by the user on an "OK" option in the prompt information 414, the electronic device 200 may display the interfaces shown in FIG. 4G to FIG. 4I to prompt the user to start to collect the audio data.

After the wearable device 100 stops collection of the audio data, if the wearable device 100 determines that previously collected audio data is valid, the wearable device 100 may send a message to the electronic device 200. The message indicates the electronic device 200 to start to notify the user to collect the physiological data of the user.

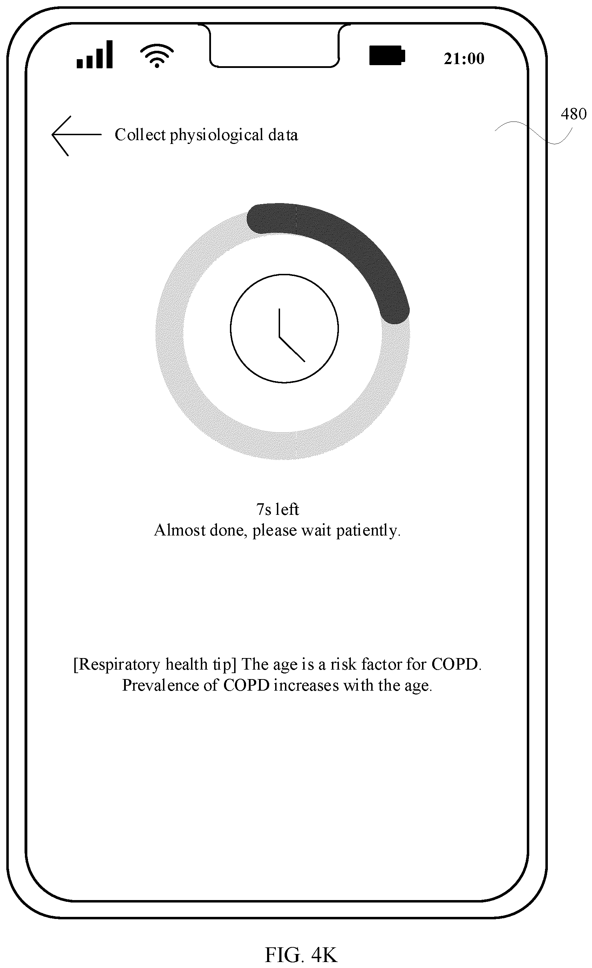

In response to the message sent by the wearable device 100, the electronic device 200 may display a user interface 480 shown in FIG. 4k. The user interface 480 is configured to notify the user to start to collect the physiological data. The user interface 480 is configured to: notify the user that the wearable device 100 is collecting the physiological data, and show collection duration of the physiological data.

Optionally, the foregoing method step of determining whether audio collected by the wearable device 100 is valid or invalid may also be performed by the electronic device 200. This is not limited in this application.

In another embodiment, the user interfaces shown in FIG. 4G to FIG. 4K may also be displayed on the wearable device 100. This is not limited in this application.

Optionally, in the process in which the wearable device 100 collects the audio data and/or the physiological data, if the wearable device 100 determines, based on motion data monitored by the motion sensor, that the user is in a moving state rather than a still state, the wearable device 100 may notify the user to maintain the still state and collect the audio data and/or the physiological data again, to avoid exerting impact, of the audio data and/or the physiological data collected in the moving state, on a detection result.

Optionally, the electronic device 200 may also detect whether the user is in the moving state or the still state. The electronic device 200 may be used to notify the user to maintain the still state and collect the audio data and/or the physiological data again. This is not limited in this application.

**The wearable device 100 may send the audio data and the physiological data to the electronic device 200 after the wearable device 100 collects the audio data and the physiological data.**

In a possible implementation, the wearable device 100 may first send the audio data to the electronic device 200 after the wearable device 100 collects the audio data. The wearable device 100 sends the physiological data to the electronic device 200 after the wearable device 100 collects the physiological data.

In another possible implementation, the wearable device 100 may simultaneously send the audio data and the physiological data to the electronic device 200 after obtaining the audio data and the physiological data.

After the electronic device 200 obtains the audio data and the physiological data, the electronic device 200 may obtain the pulmonary function detection data of the user based on the audio data and the physiological data. Optionally, the wearable device 100 may obtain the pulmonary function detection data of the user based on the audio data and the physiological data, and then send the pulmonary function detection data of the user to the electronic device 200, so that the electronic device 200 obtains the pulmonary function detection data of the user.

**The electronic device 200 may obtain pulmonary function standard data, and compare the pulmonary function detection data and the pulmonary function standard data for display, so that the user can intuitively view whether the pulmonary function detection data of the user falls within a normal range.**

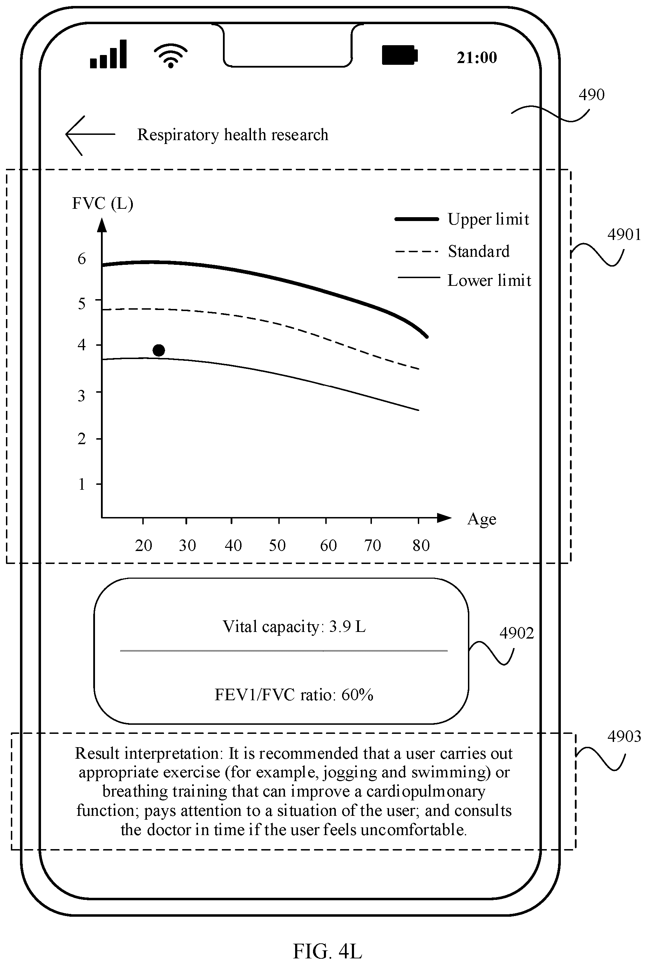

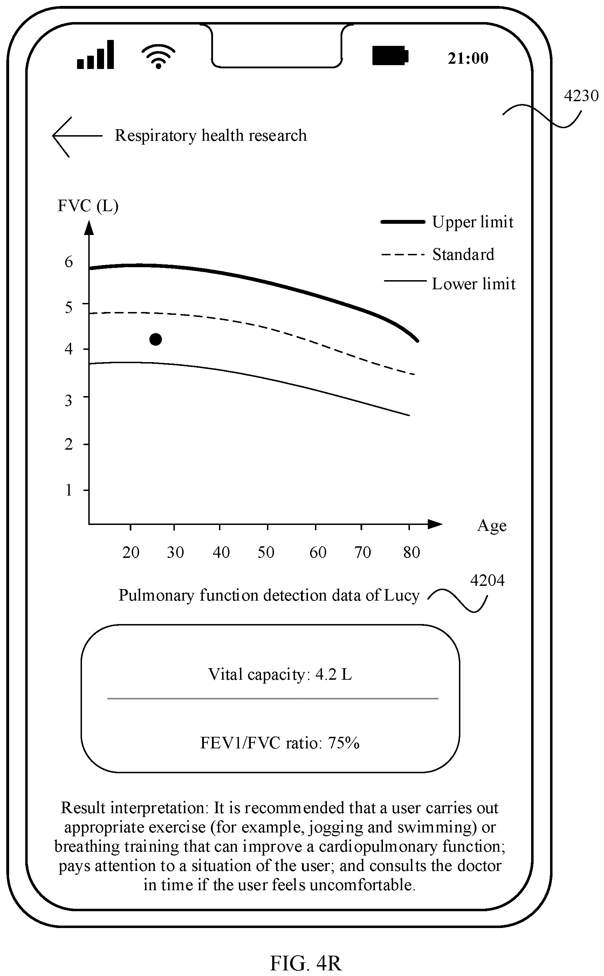

The pulmonary function standard data may be obtained based on a gender and a height of a detected user, or may be obtained only based on a gender of the user. The pulmonary function standard data may be standard vital capacity data of users of different heights at different ages. The electronic device 200 may display the pulmonary function standard data in a graph, so that the user can view a change trend of the pulmonary function standard data. For example, the pulmonary function standard data may be a curve of a correspondence between a standard vital capacity and an age shown in FIG. 4L to FIG. 4N, or FIG. 4R.

For example, the electronic device 200 may display a user interface 490 shown in FIG. 4L. The user interface 490 may include a display area 4901, a display area 4902, and a display area 4903.

The display area 4901 may include a graph. The graph includes the curve of the correspondence between a standard vital capacity and an age and a real-time vital capacity. That the real-time vital capacity is 3.9 L is shown in the display area 4901. The curve of the correspondence between a standard vital capacity and an age may include a standard curve, an upper limit curve, and a lower limit curve. If the real-time vital capacity is close to the standard curve, it indicates that a vital capacity of the user is normal. If the real-time vital capacity is close to or below the lower limit curve, it indicates that the vital capacity of the user is too small and does not meet a standard. If the real-time vital capacity is close to or above the upper limit curve, it indicates that the vital capacity of the user is good and exceeds the standard.

In some embodiments, the electronic device 200 may not display the standard curve or the upper limit curve.

The display area 4902 includes the pulmonary function standard data. For example, the vital capacity is 3.9 L, and a forced expiratory volume in 1 second/forced vital capacity ratio is 60%.

The user may learn, from the display area 4901, whether the real-time vital capacity of the user is normal or abnormal. If the real-time vital capacity is close to or below the lower limit curve, it can be considered that the real-time vital capacity of the user is abnormal. If the real-time vital capacity is higher than the lower limit curve by a specified distance, it can be considered that the real-time vital capacity of the user is normal.

The display area 4903 includes a targeted suggestion given for the detected pulmonary function detection data of the user. For example, the suggestion may be "It is recommended that a user carries out appropriate exercise (for example, jogging and swimming) or breathing training that can improve a cardiopulmonary function; pays attention to a situation of the user; and consults the doctor in time if the user feels uncomfortable".

The standard curve, the upper limit curve, and the lower limit curve shown in FIG. 4L may be related to detection of the height of the user.

The standard curve, the upper limit curve, and the lower limit curve may be determined based on the detected height of the user.

For example, the height of the detected user is a first height. The standard curve may be a curve of standard vital capacities of users of the first height at different ages. The upper limit curve may be a curve of standard vital capacities of users of a second height at different ages. The lower limit curve may be a curve of standard vital capacities of users of a third height at different ages. The second height is equal to the first height plus a first threshold, and the third height is equal to the first height minus a second threshold. The second threshold may be the same as or different from the first threshold.

For example, the height of the detected user may be 175 centimeters. In this case, the standard curve may be a curve of standard vital capacities of users of 175 centimeters at different ages. The upper limit curve may be a curve of standard vital capacities of users of 180 centimeters at different ages. The lower limit curve may be a curve of standard vital capacities of users of 170 centimeters at different ages. Both the second threshold and the first threshold are 5 cm.

For example, the height of the detected user is a first height. The standard curve may be a curve of standard vital capacities of users of the first height at different ages. The upper limit curve may be a curve of standard vital capacities of users of a maximum height at different ages. The lower limit curve may be a curve of standard vital capacities of users of a minimum height at different ages.

For example, the height of the detected user may be 175 centimeters. In this case, the standard curve may be a curve of standard vital capacities of users of 175 centimeters at different ages. The upper limit curve may be a curve of standard vital capacities of users of 190 centimeters at different ages. The lower limit curve may be a curve of standard vital capacities of users of 160 centimeters at different ages. 190 centimeters is the maximum height of the user, and 160 centimeters is the minimum height of the user.

The standard curve, the upper limit curve, and the lower limit curve shown in FIG. 4L may alternatively be unrelated to the height of the detected user, and related to an average height of a population.

For example, an average age of the population is the first height. The standard curve may be a curve of standard vital capacities of users of the first height at different ages. The upper limit curve may be a curve of standard vital capacities of users of a maximum height at different ages. The lower limit curve may be a curve of standard vital capacities of users of a minimum height at different ages.

For example, an average age of the population may be 175 centimeters. In this case, the standard curve may be a curve of standard vital capacities of users of 175 centimeters at different ages. The upper limit curve may be a curve of standard vital capacities of users of 190 centimeters at different ages. The lower limit curve may be a curve of standard vital capacities of users of 160 centimeters at different ages. 190 centimeters is the maximum height of the user, and 160 centimeters is the minimum height of the user.

It should be noted that FIG. 4L may further include more other curves of the standard vital capacity, which are not limited to the standard curve, the upper limit curve, and the lower limit curve. This is not limited in this application.

It should be noted that a manner of determining the pulmonary function standard data may be related to the gender. Because there is a large difference between a vital capacity of a male and a vital capacity of a female, before the pulmonary function standard data is shown in FIG. 4L, the electronic device 200 may first obtain the gender of the detected user, or the gender and the height of the detected user, and determine the pulmonary function standard data based on the gender of the user, or the gender and the height of the detected user.

In some embodiments, if the electronic device 200 does not obtain the gender of the detected user, or the gender and the height of the detected user, the electronic device 200 may notify the user to enter the gender of the detected user, or the gender and the height of the detected user.

In some embodiments, if the electronic device 200 obtains the height of the detected user, but does not obtain the gender of the detected user, in a possible implementation, the electronic device 200 may notify the user to enter the gender of the detected user; in another possible implementation, the electronic device 200 may alternatively predict the gender of the detected user based on the obtained height of the detected user. Specifically, an average height of males and an average height of females are different. The electronic device 200 may obtain a first height threshold based on the average height of the males and the average height of the females. When the height of the detected user is greater than the first height threshold, the electronic device 200 may predict that the gender of the detected user is a male. When the height of the detected user is less than the first height threshold, the electronic device 200 may predict that the gender of the detected user is a female. Then, the electronic device 200 determines the pulmonary function standard data based on the predicted gender of the detected user, or the predicted gender of the detected user and the predicted height of the detected user.

**Optionally, the user may also switch graphs of different styles displayed in the display area 4901. The real-time vital capacity of the user may be displayed by using the graphs of the different styles.**

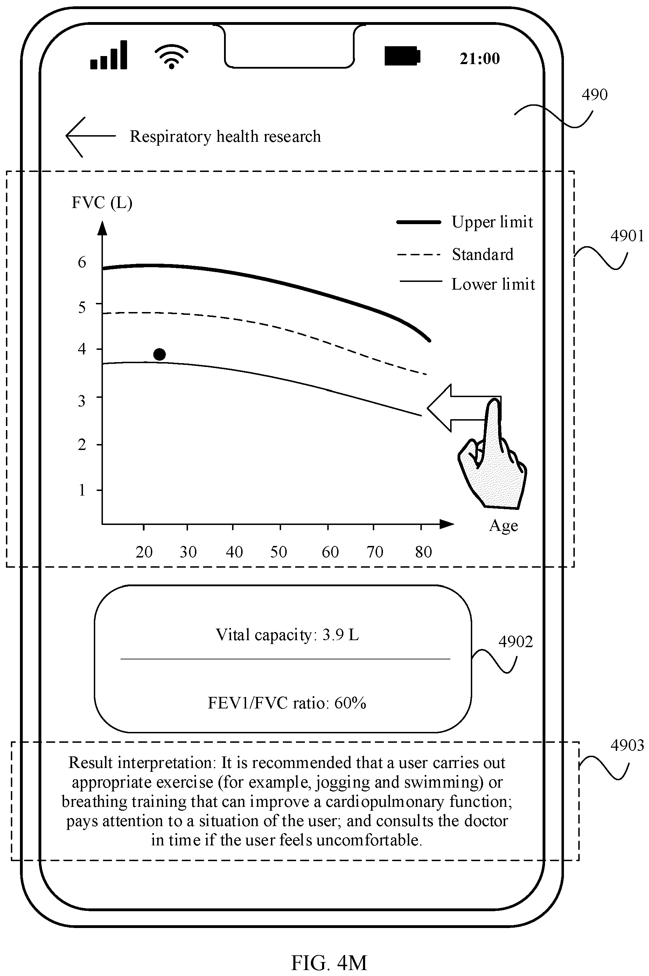

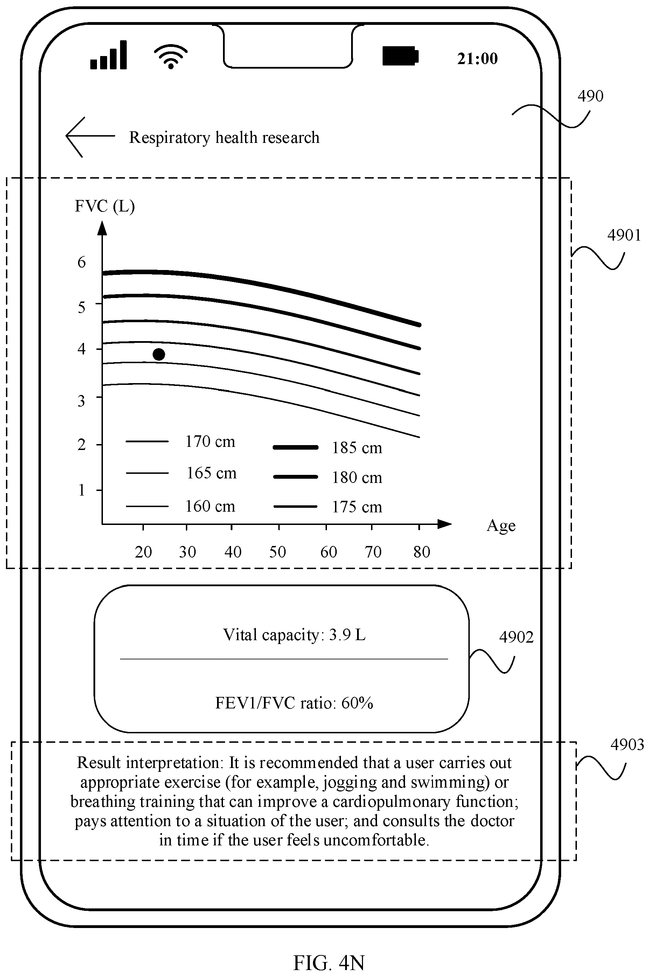

For example, as shown in FIG. 4M, the electronic device 200 may receive a slide operation (for example, a leftward slide operation) performed by the user in the display area 4901. In response to the slide operation performed by the user, the electronic device 200 may switch to display a graph of another style in the display area 4901. For example, the electronic device 200 may display a graph shown in FIG. 4N.

The graph shown in FIG. 4N includes a curve of a correspondence between a standard vital capacity and an age and a real-time vital capacity table. A difference between FIG. 4N and FIG. 4M lies in that the graph shown in FIG. 4N includes a plurality of curves, and different curves correspond to correspondences between a same height at different ages and a standard vital capacity, for example, a curve of standard vital capacities of users of a height of 160 centimeters at different ages, a curve of standard vital capacities of users of a height of 165 centimeters at different ages, a curve of standard vital capacities of users of a height of 170 centimeters at different ages, a curve of standard vital capacities of users of a height of 175 centimeters at different ages, a curve of standard vital capacities of users of a height of 180 centimeters at different ages, and a curve of standard vital capacities of users of a height of 185 centimeters at different ages.

FIG. 4N may further include a curve of standard vital capacities of users of another height at different ages. FIG. 4N sets no limitation.

The user may learn a curve of a standard vital capacity corresponding to the height of the detected user from the display area 4901. If the real-time vital capacity falls within a specific range around the curve of the standard vital capacity corresponding to the height of the detected user, it may be considered that the vital capacity of the user is normal. If the real-time vital capacity exceeds the specific range around the curve of the standard vital capacity corresponding to the height of the detected user, it may be considered that the vital capacity of the user is abnormal.

For example, if the height of the detected user is 175 centimeters, the real-time vital capacity of the user is 3.9 L. It can be learned from FIG. 4N that the real-time vital capacity of the detected user is low, and is abnormal.

It should be noted that the curve of the correspondence between a standard vital capacity and an age shown in FIG. 4N may be related to the gender. Because there is a large difference between a vital capacity of a male and a vital capacity of a female, before FIG. 4N shows the curve of the correspondence between a standard vital capacity and an age, the electronic device 200 may first obtain the gender of the detected user, and determine the curve of the correspondence between a standard vital capacity and an age based on the gender of the detected user.

In some embodiments, if the electronic device 200 does not obtain the gender of the detected user, the electronic device 200 may notify the user to enter the gender of the detected user.

It should be noted that this application is described only by using the vital capacity and the forced expiratory volume in 1 second/forced vital capacity ratio as examples. More other pulmonary function detection data may be further displayed in a graph. This is not limited in this application.

**In some implementations, the user not only may detect the pulmonary function of the user by using the electronic device 200, but also may detect a pulmonary function of another user (for example, a family member) by using the electronic device 200. In addition, detection data of different users is stored separately. In this way, the user may query pulmonary function detection data of different users in specific duration.**

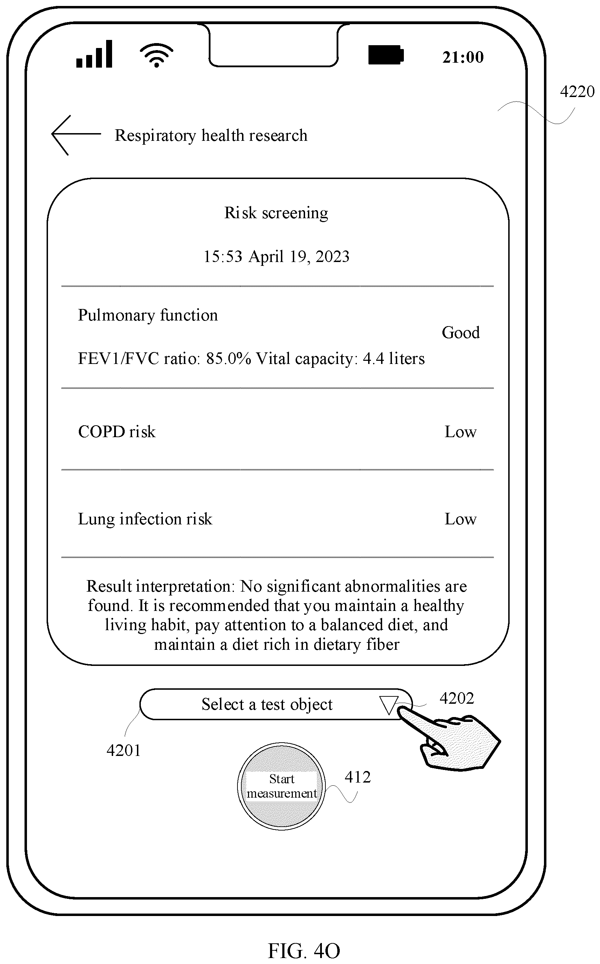

FIG. 4O to FIG. 4R are diagrams in which an electronic device 200 detects pulmonary functions of different users.

As shown in FIG. 4O, before the electronic device 200 receives a user operation to start to detect the pulmonary function, the electronic device 200 may display a user interface 4220. The user interface 4220 is similar to the user interface 420. A difference lies in that the user interface 4220 further includes an icon 4201, and the icon 4201 is used by the user to select a detection object.

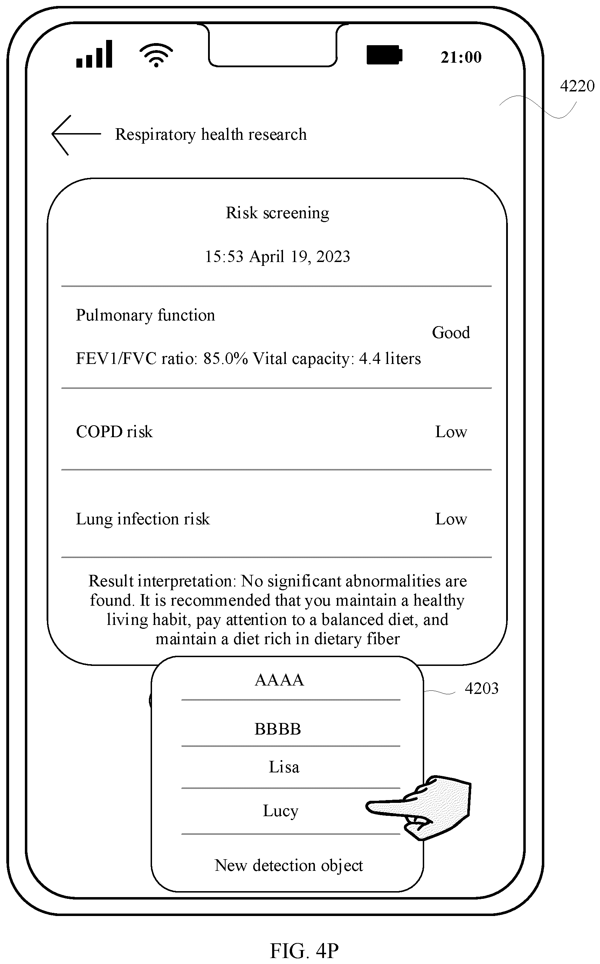

As shown in FIG. 4O, the electronic device 200 may receive an input operation (for example, a single-tap) performed by the user on an option 4202 in the icon 4201, and in response to the input operation performed by the user, the electronic device 200 may display a selection bar 4203 shown in FIG. 4P. Options of a plurality of detection objects are shown in the selection bar 4203. For example, the plurality of detection objects include but are not limited to a detection object "AAAA", a detection object "BBBB", a detection object "Lisa", and a detection object "Lucy". The user may start to detect a pulmonary function after selecting any detection object, and bind current detection data and a currently selected detection object for storage.

Optionally, if the selection bar 4203 does not include an option of a user who needs to be detected currently, the electronic device 200 may receive an input operation performed by the user on an option of "New detection object" in the selection bar 4203, and add the option of the user who needs to be detected currently.

In this way, detection data of different detection objects may be stored separately, to help subsequently view the detection data of the different detection objects in specific duration.

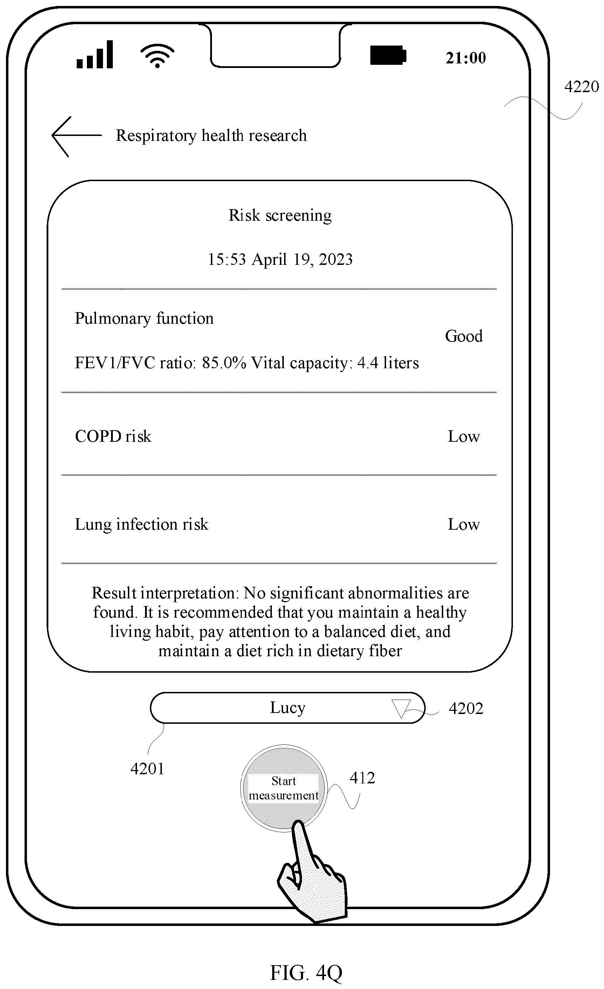

For example, as shown in FIG. 4P, the electronic device 200 may receive an input operation (for example, a single-tap) performed by the user on the option of the detection object "Lucy" in the selection bar 4203, and in response to the input operation performed by the user, the electronic device 200 may determine that a current detection object is "Lucy", and the electronic device 200 may display, in the icon 4201, a "Lucy" identifier shown in FIG. 4Q.

Then, the electronic device 200 may receive an input operation (for example, a single-tap) performed by the user on the control 412, and in response to the input operation performed by the user may start to measure the pulmonary function detection data.

After the electronic device 200 obtains pulmonary function detection data of a currently detected user, the electronic device 200 may display a user interface 4230 shown in FIG. 4R. The user interface 4230 is similar to the user interface 490. A difference lies in that the user interface 4230 further includes prompt information 4204, and content of the prompt information 4204 may include "Pulmonary function detection data of Lucy". In this way, the user may learn, from the user interface 4230, that a current detection object of the pulmonary function is "Lucy".

Then, the electronic device 200 may bind the pulmonary function detection data of the detection object "Lucy" and the detection object "Lucy" for storage. Specifically, the electronic device 200 may find a storage area of the detection object "Lucy", and store the pulmonary function detection data of the detection object "Lucy" in the storage area of the detection object "Lucy". It should be noted that storage areas of different detection objects are different and are isolated from each other.

For pulmonary function standard data shown in FIG. 4L to FIG. 4N and FIG. 4R, only a vital capacity in the pulmonary function standard data is shown in a graph. In another embodiment, the forced expiratory volume in 1 second/forced vital capacity ratio in the function standard data may also be shown in a graph, or both the vital capacity and the forced expiratory volume in 1 second/forced vital capacity ratio in the pulmonary function standard data may be shown in a graph. This is not limited in this application.

**The pulmonary function standard data shown in FIG. 4L to FIG. 4N and FIG. 4R may be obtained based on empirical data, or may be obtained based on statistical data, or may be obtained by combining the empirical data and the statistical data.**

The empirical data may be obtained based on the height and the age according to an estimated value formula.

$$\text{Male standard vital capacity}=\exp[0.0576*(\text{HEIGHT}*100)-0.026*\text{AGE}-4.34]$$

Formula 1

$$\text{Female vital capacity}=\exp[0.0443*(\text{HEIGHT}*100)-0.026*\text{AGE}-2.89] \quad \text{Formula 2}$$

Formula 1 and Formula 2 show correspondences between a standard vital capacity and both of a height and an age of users of different genders.

In Formula 1 and Formula 2, exp represents an exponentiation operation in which a base e of a natural logarithm is used as a base, HEIGHT represents a height, and AGE represents an age. Standard vital capacities corresponding to users of different genders at different ages and different heights may be obtained based on Formula 1 and Formula 2.

It should be noted that the parameters in Formula 1 and Formula 2 may alternatively have other values. This application merely provides example descriptions, and does not constitute a limitation.

$$\text{Male forced expiratory volume in 1 second/forced vital capacity ratio} = \exp[0.7403 - 0.1595 * \ln(\text{HEIGHT} * 100) - 0.0366 * \ln(\text{AGE}) + 0.1693 - 0.8394 * (\text{AGE}/100) + 2.3953 * (\text{AGE}/100)^2 - 3.8845 * (\text{AGE}/100)^3 + 2.9921 * (\text{AGE}/100)^4 - 0.8703 * (\text{AGE}/100)^5] \quad \text{Formula 3}$$

$$\text{Female forced expiratory volume in 1 second/forced vital capacity ratio} = \exp[0.5506 - 0.1078 * \ln(\text{HEIGHT} * 100) - 0.0544 * \ln(\text{AGE}) + 0.0590 - 0.4177 * (\text{AGE}/100) - 2.9918 * (\text{AGE}/100)^2 - 6.7192 * (\text{AGE}/100)^3 + 6.5489 * (\text{AGE}/100)^4 - 2.3439 * (\text{AGE}/100)^5] \quad \text{Formula 4}$$

Formula 3 and Formula 4 show correspondences between a forced expiratory volume in 1 second/forced vital capacity ratio and both of a height and an age of users of different genders.

In Formula 3 and Formula 4, exp represents an exponentiation operation in which a base e of a natural logarithm is used as a base, ln represents a logarithmic operation in which a base e of a natural logarithm is used as a base, HEIGHT represents a height, and AGE represents an age. Forced expiratory volume in 1 second/forced vital capacity ratios corresponding to users of different genders at different ages and different heights may be obtained based on Formula 3 and Formula 4.

It should be noted that the parameters in Formula 3 and Formula 4 may alternatively have other values. This application merely provides example descriptions, and does not constitute a limitation.

Standard vital capacities and forced expiratory volume in 1 second/forced vital capacity ratios corresponding to users of different genders at different ages and different heights may be obtained based on Formula 1, Formula 2, Formula 3, and Formula 4.

In some embodiments, the pulmonary function standard data may alternatively be obtained based on the statistical data.

A server may collect a large amount of data, to obtain the statistical data, and perform statistical analysis on the statistical data, to obtain the pulmonary function standard data. The statistical data collected by the server may be reported by a terminal device, or may be obtained from a pulmonary function testing institution.

For example, after the user detects the pulmonary function on the terminal device and obtains the pulmonary function detection data, the terminal device may report the pulmonary function detection data to the server after the user grants authorization, and the server may obtain pulmonary function detection data of a large quantity of users.

For another example, the testing institution may detect the pulmonary function of the user by using a professional instrument. After obtaining authorization of the testing institution and the detected user, the testing institution may report pulmonary function detection data of a large quantity of users to the server.

It should be noted that the pulmonary function detection data may alternatively be obtained in another manner. This is not limited in this application.

In addition to the pulmonary function detection data and/or the forced expiratory volume in 1 second/forced vital capacity ratio, the pulmonary function detection data collected by the server may further include information such as the gender, the age, and the height of the detected user.

After the server obtains a large amount of pulmonary function detection data, the server may divide the pulmonary function detection data into male pulmonary function detection data and female pulmonary function detection data. In addition, pulmonary function detection data of different genders is analyzed based on information such as ages and heights of users of different genders, to obtain pulmonary function standard data corresponding to the users of different genders at different heights and ages.

According to the foregoing descriptions, the server may update the pulmonary function standard data periodically/irregularly/at a specific interval. Because the pulmonary function standard data is obtained based on actual data, the pulmonary function standard data is more accurate.

In another embodiment, after the server obtains pulmonary function standard data through updating based on the statistical data, the server may also combine the pulmonary function standard data obtained through updating based on the statistical data and the pulmonary function standard data obtained based on the empirical data, to obtain the pulmonary function standard data. In this way, the empirical data and the statistical data are combined to obtain the standard data of pulmonary function, which better matches an actual normal range and a comparison result.

The server may further obtain the pulmonary function standard data in another manner. This is not limited in this application.

After obtaining the pulmonary function standard data, the server may send the pulmonary function standard data to the electronic device 200, so that the electronic device 200 can display the pulmonary function standard data in an icon.

**In some embodiments, the electronic device 200 may store pulmonary function detection data in first duration before current time. Pulmonary function detection data that exceeds the first duration is deleted, to save storage space of the electronic device 200.**

**In some embodiments, the electronic device 200 may alternatively store pulmonary function detection data at a preset quantity of detection times, to save storage space of the electronic device 200. For example, the electronic device 200 may store pulmonary function detection data at ten times. When detecting an eleventh time, the electronic device 200 may delete pulmonary function detection data at a first time.**

**Optionally, the electronic device 200 may also receive a user operation to view the pulmonary function detection data in the first duration.**

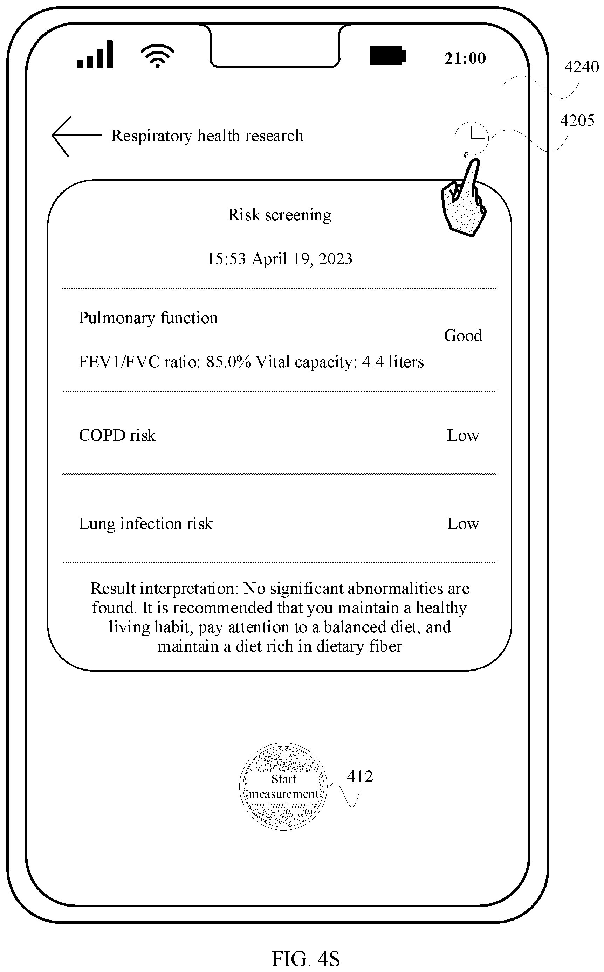

**FIG. 4S to FIG. 4V are diagrams in which an electronic device 200 displays pulmonary function detection data in first duration.**

As shown in FIG. 4S, before the electronic device 200 receives a user operation to start to detect the pulmonary function, the electronic device 200 may display a user interface 4240. The user interface 4240 is similar to the user interface 420. A difference lies in that the user interface 4240 further includes an icon 4205, and the icon 4205 is configured to display the pulmonary function detection data in the first duration.

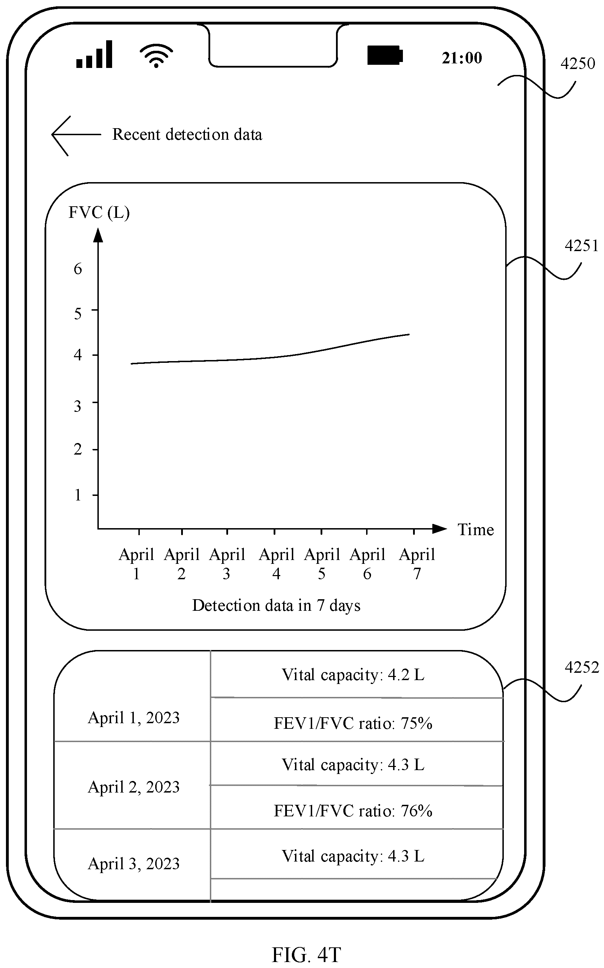

As shown in FIG. 4S, the electronic device 200 may receive an input operation (for example, a single-tap) performed by the user on the icon 4205 in the user interface 4240, and in response to the input operation performed by the user, the electronic device 200 may obtain the pulmonary function detection data of the user in the first duration, and display a user interface 4250 shown in FIG. 4T.

The user interface 4250 includes a graphic display area 4251 of the pulmonary function detection data in the first duration and a data display area 4252 of the pulmonary function detection data in the first duration. The data display area 4252 includes pulmonary function detection data at different dates. For example, on April 1, 2023, a vital capacity detected by the user is 4.2 L, and a forced expiratory volume in 1 second/forced vital capacity ratio is 75%; on April 2, 2023, a vital capacity detected by the user is 4.3 L, and a forced expiratory volume in 1 second/forced vital capacity ratio is 76%; and On April 1, 2023, a vital capacity detected by the user is 4.3 L. The user may slide downward to view pulmonary function detection data at more other dates.

The user may intuitively view a change trend of the pulmonary function detection data of the user in the first duration in the graphic display area 4251.

In some embodiments, the electronic device 200 may also receive a user operation to view pulmonary function detection data of different users.

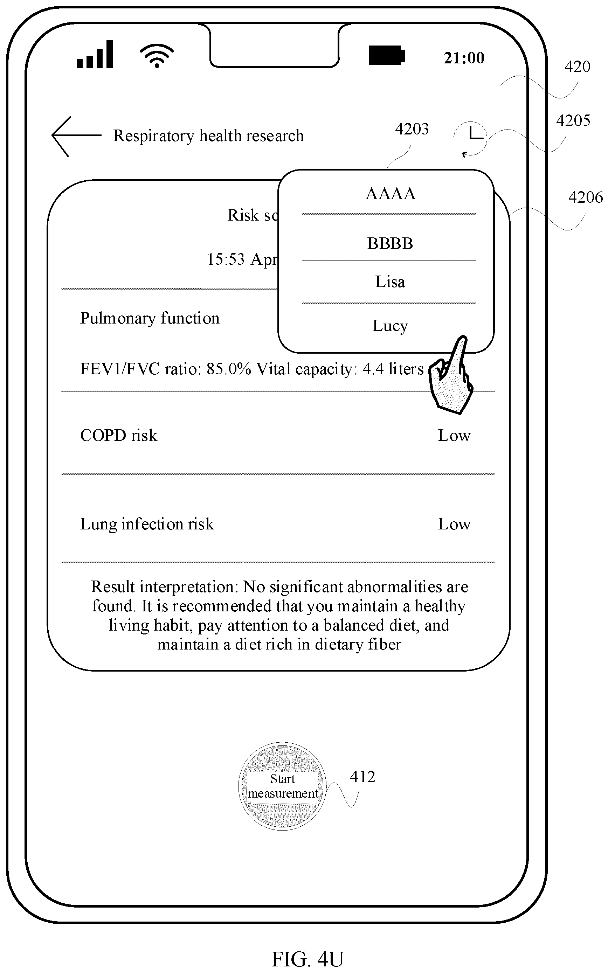

With reference to the descriptions in FIG. 4S, the electronic device 200 may receive the input operation performed by the user on the icon 4205 in the user interface 4240, and in response to the input operation performed by the user, the electronic device 200 may display a selection bar 4206 shown in FIG. 4U. Options of a plurality of different detection objects are shown in the selection bar 4206. For example, the plurality of detection objects include but are not limited to a detection object "AAAA", a detection object "BBBB", a detection object "Lisa", and a detection object "Lucy". The user may start to view pulmonary function detection data of any detection object in the first duration after selecting the detection object.

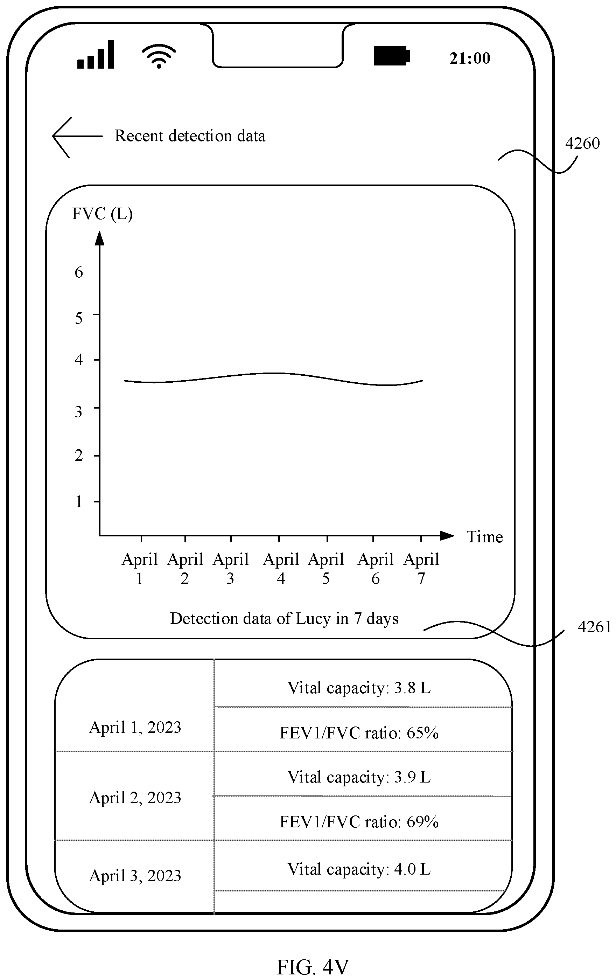

As shown in FIG. 4U, the electronic device 200 may receive an input operation (for example, a single-tap) performed by the user on the option of the detection object "Lucy" in the selection bar 4206, and in response to the input operation performed by the user, the electronic device 200 may obtain pulmonary function detection data of the detection object "Lucy" in the first duration, and display a user interface 4260 shown in FIG. 4V. The user interface 4260 is similar to the user interface 4250. A difference lies in that the user interface 4260 includes prompt information "Detection data of Lucy in seven days", to notify that the interface shows detection data of the detection object "Lucy" in specific duration.

According to the method, the electronic device 200 not only may display pulmonary function detection data of a current user, but also may display pulmonary function detection data of another user.

In some embodiments, the pulmonary function detection data that is of different users in the first duration and that is stored in the electronic device 200 may be pulmonary function detection data that is of the different users in the first duration and that is collected by the wearable device 100, sent to the electronic device 200, and stored in the electronic device 200.

In another embodiment, the pulmonary function detection data that is of different users in the first duration and that is stored in the electronic device 200 may be sent by another electronic device to the electronic device 200 periodically/irregularly/at a specific interval. After a user of the another electronic device is authorized by the user, the another electronic device may send stored pulmonary function detection data of the user to the electronic device 200 periodically/irregularly/at a specific interval, so that the electronic device 200 can store the pulmonary function detection data of the different users in the first duration, to help the user using the electronic device 200 view pulmonary function detection data of another authorized user in the first duration. For example, the another authorized user may be a family member of the user using the electronic device 200. For example, the another authorized user may be a parent, a child, or the like of the user using the electronic device 200. Therefore, after the electronic device 200 obtains pulmonary function detection data of the parent or the child in the first duration, the user using the electronic device 200 may view the pulmonary function detection data of the another authorized user in the first duration, to help monitor a pulmonary function of the family member.

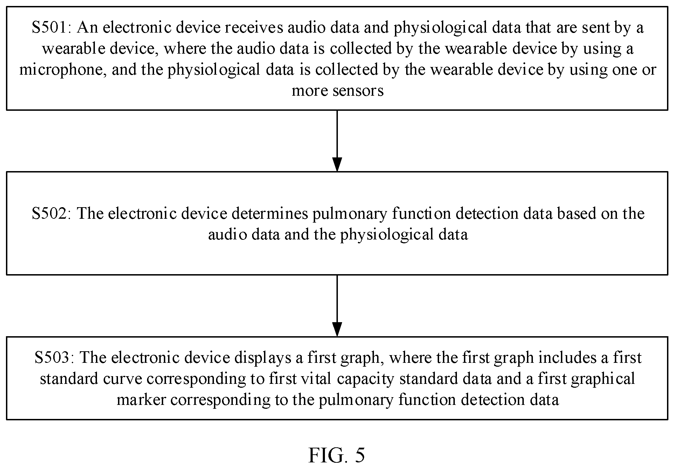

FIG. 5 is a schematic flowchart of a health data display method according to this application.

S501: An electronic device receives audio data and physiological data that are sent by a wearable device, where the audio data is collected by the wearable device by using a microphone, and the physiological data is collected by the wearable device by using one or more sensors.

S502: The electronic device determines pulmonary function detection data based on the audio data and the physiological data.

The pulmonary function detection data includes any one or more of the following: a vital capacity, a forced expiratory volume in 1 second/forced vital capacity ratio, a forced expiratory volume in 1 second, peak expiratory flow, peak inspiratory flow, maximal expiratory pressure, maximal inspiratory pressure, maximal voluntary ventilation, and maximal mid-expiratory flow.

The forced expiratory volume in 1 second/forced vital capacity ratio indicates a percentage of an amount of air exhaled in a first second to a total amount of air exhaled in effective duration.

The forced expiratory volume in 1 second indicates a total amount of air exhaled in the first second.

The peak expiratory flow indicates an instantaneous maximum value of exhaled air flow in a maximum forced exhalation process.

The peak inspiratory flow indicates an instantaneous maximum value of inhaled air flow in a maximum forced inhalation process.

The maximal expiratory pressure indicates a maximum value of oral pressure capable of being generated in the maximum forced exhalation process.

The maximal inspiratory pressure indicates a maximum value of oral pressure capable of being generated in the maximum forced inhalation process.

The maximal voluntary ventilation indicates maximum ventilation in 1 minute.

The maximal mid-expiratory flow indicates a mean expiratory flow rate when 25% to 75% of the vital capacity is exhaled forcibly.

S503: The electronic device displays a first graph, where the first graph includes a first standard curve corresponding to first vital capacity standard data and a first graphical marker corresponding to the pulmonary function detection data.

The first vital capacity standard data may be pre-stored in the device by the electronic device, or the first vital capacity standard data may be obtained by the electronic device from a server in real time or obtained from another device.

In a possible implementation, the first vital capacity standard data is related to a first gender of a first user and a first height of the first user, and the first standard curve includes a first curve, a second curve, and a third curve; the first curve includes a curve of a change in vital capacity data of a user group of the first gender at the first height with an age; the second curve includes a curve of a change in vital capacity data of the user group of the first gender at a second height with the age; the third curve includes a curve of a change in vital capacity data of the user group of the first gender at a third height with the age; and the second height is greater than the first height, and the third height is less than the first height.

For example, the first curve may be a standard curve shown in FIG. 4L.

The second curve may be an upper limit curve shown in FIG. 4L.

The third curve may be a lower limit curve shown in FIG. 4L.

The first graphical marker may be a graphical marker (for example, a solid circle) shown in FIG. 4L.

Herein, the first vital capacity standard data is related to a gender and a height of a detected user (the first user). Users of different genders and different heights correspond to different first vital capacity standard data.

According to the first aspect, in a possible implementation, the second height includes an average minimum height of the user group of the first gender, and the third height is an average maximum height of the user group of the first gender.

Herein, a generation manner of obtaining the second curve and the third curve is shown. The generation manner of the second curve and the third curve is unrelated to the first height of the first user, and is related to the first gender of the first user.

In a possible implementation, a difference between the first height and the second height is a first threshold, and a difference between the third height and the first height is a second threshold; and the first threshold is the same as or different from the second threshold.

Herein, another generation manner of obtaining the second curve and the third curve is shown. The generation manner of the second curve and the third curve is related to the first height of the first user.

In a possible implementation, after the electronic device displays the first graph, the method further includes: The electronic device receives a first operation performed by a user. The electronic device obtains second vital capacity standard data in response to the first operation. The electronic device displays a second graph. The second graph includes a second standard curve corresponding to the second vital capacity standard data and a second graphical marker corresponding to the pulmonary function detection data, and the second vital capacity standard data is related to the first gender of the first user. The second standard curve includes a plurality of curves of changes in vital capacity data of the user group of the first gender at different heights with the age.

For example, the first operation may be a slide operation (for example, a leftward slide operation) in a display area 4901 shown in FIG. 4M.

The second standard curve may be a plurality of curves shown in FIG. 4N.

The second vital capacity standard data is different from the first vital capacity standard data. The second vital capacity standard data may be pre-stored in the device by the electronic device, or the second vital capacity standard data may be obtained by the electronic device from the server in real time or obtained from another device.

The electronic device may display the second vital capacity standard data by using a plurality of curves. The plurality of curves may be related to the first gender of the first user, and unrelated to the first height of the first user.

In another embodiment, before the electronic device displays the pulmonary function detection data, the electronic device may also notify the user to select one piece of pulmonary function standard data from a plurality of pieces of pulmonary function standard data, and compare, for display, the pulmonary function standard data selected by the user and the pulmonary function detection data.

In this implementation, the electronic device may receive a user operation to obtain different pulmonary function standard data, and compare the pulmonary function detection data and the different pulmonary function standard data for display. That is, the pulmonary function detection data of the detected user is displayed by switching graphs of different styles.

In a possible implementation, before the electronic device displays the first graph, the method further includes: The electronic device displays a first user interface. The first user interface further includes first prompt information, the first prompt information includes identifiers of a plurality of users, and the identifiers of the plurality of users include an identifier of the first user. The electronic device receives a first selection operation performed by the user on the identifier of the first user in the first user interface. The electronic device obtains the first vital capacity standard data after receiving the selection operation performed on the identifier of the first user.

For example, the first user interface may be a user interface 4220 shown in FIG. 4O.

The first prompt information may be a selection bar 4203 shown in FIG. 4O, and the identifiers of the plurality of users may be options of a plurality of detection objects shown in the selection bar 4203.

The first selection operation may be an input operation (for example, a single-tap) performed on an option of a detection object "Lucy" in the selection bar 4203.

The identifier of the first user may be the option of the detection object "Lucy".

In this implementation, the electronic device may detect pulmonary functions of different users, for example, detect pulmonary functions of different family members. The first graph is related to the gender and the height of the detected user or the gender of the detected user. If different detected users have different genders and heights or different detected users have different genders, the electronic device also obtains different first standard curves. Therefore, before displaying the first graph, the electronic device may notify the user to select or add the detected user, to determine the gender and the height of the detected user or the gender of the detected user.

Optionally, if the electronic device does not store a gender and a height of a currently detected user or a gender of a currently detected user, the electronic device may further notify the user to enter the gender and the height of the currently detected user or the gender of the currently detected user.

In a possible implementation, after the electronic device determines the pulmonary function detection data based on the audio data and the physiological data, the method further includes: The electronic device stores the pulmonary function detection data in a storage area corresponding to the first user. Different users correspond to different storage areas.

In this implementation, pulmonary function detection data of different detected users is stored in different storage areas, to facilitate subsequent obtaining and viewing of the pulmonary function detection data of the different users.

In a possible implementation, the first user interface further includes a first control, and before the electronic device receives audio data and physiological data that are sent by a wearable device, the method further includes: The electronic device receives a second operation performed by the user on the first control. The electronic device sends a first message to the wearable device in response to the second operation. The first message indicates the wearable device to collect the audio data and the physiological data.

For example, the first control may be an "OK" control shown in FIG. 4C.

The first user interface may be a user interface 430 shown in FIG. 4C.

The second operation may be an input operation (for example, a single-tap) performed on the "OK" control in the user interface 430 shown in FIG. 4C.

In this way, before starting to detect the pulmonary function, the electronic device may send a message to the wearable device after receiving a detection start operation of the user, so that the wearable device starts to collect audio data and physiological data of the detected user after receiving the message sent by the electronic device.

In a possible implementation, after the electronic device displays the first graph, the method further includes: The electronic device displays a second user interface. A second control is displayed in the second user interface. The electronic device receives a third operation performed by the user on the second control. The electronic device displays a third graph in response to the third operation. The third graph includes pulmonary function detection data in a first time period.

For example, the second user interface may be a user interface 4240 shown in FIG. 4S.

The second control may be an icon 4205 shown in FIG. 4S.

The third operation may be an input operation performed on the icon 4205 in the user interface 4240.

The third graph may be a graph shown in FIG. 4T.

In this way, the electronic device may store pulmonary function detection data of the detected user in specific duration, and the electronic device may also receive a user operation to view the pulmonary function detection data of the detected user in the specific duration.

In a possible implementation, the second user interface further includes second prompt information, the second prompt information includes the identifiers of the plurality of users, and the identifiers of the plurality of users include the identifier of the first user; and before the electronic device displays the third graph, the method further includes: The electronic device receives a second selection operation performed by the user on the identifier of the first user in the second user interface. The electronic device obtains pulmonary function detection data of the first user in the first time period based on the identifier of the first user in response to the second selection operation.

For example, the second prompt information may be a selection bar 4206 shown in FIG. 4U.

The identifier of the first user may be an option of the detection object "Lucy" shown in FIG. 4U.

The second selection operation may be an input operation (for example, a single-tap) performed on the option of the detection object "Lucy" in the selection bar 4206 shown in FIG. 4U.

The first user may be the detection object "Lucy".

The third graph may be a graph shown in FIG. 4V.

The electronic device stores pulmonary function detection data of different users in specific duration. When the electronic device receives a user operation to view the pulmonary function detection data of the user in the specific duration, the electronic device may notify the user to enter an identifier of the detected user, and obtain the pulmonary function detection data of the detected user in the specific duration from pulmonary function detection data of a plurality of detected users based on the identifier that is of the detected user and that is entered by the user.

In a possible implementation, the audio data includes any one or more of the following: a cough sound, a blow sound, and a speaking sound; and the physiological data includes any one or more of the following: a heart rate, heart rate variability, a respiratory rate, and blood oxygen.

According to the method, the electronic device may test a pulmonary function of a user in collaboration with the wearable device, to obtain pulmonary function detection data of the user and display the pulmonary function detection data, thereby implementing convenience of a pulmonary function test. In addition, the electronic device may further compare, for display, an actual value and a theoretical value of a detected pulmonary function index in a graph, so that the user can intuitively observe whether the detected pulmonary function index falls within a normal range.

Implementations of this application may be randomly combined, to achieve different technical effects.

A person of ordinary skill in the art may understand that all or some procedures of the methods in embodiments may be implemented by a computer program instructing related hardware. The program may be stored in the computer-readable storage medium. When the program is executed, the procedures in the method embodiments may be performed. The storage medium includes any medium that can store program code such as a ROM, a random access memory RAM, a magnetic disk, or a compact disc.

In conclusion, the foregoing descriptions are merely example embodiments of the technical solutions of the present invention, and are not intended to limit the protection scope of the present invention. Any modification, equivalent replacement, improvement, or the like made in accordance with the disclosure of the present invention shall be included in the protection scope of the present invention.

**Claims**

1. A health data display method, wherein the method comprises:

   receiving, by an electronic device, audio data and physiological data that are sent by a wearable device, wherein the audio data is collected by the wearable device by using a microphone, and the physiological data is collected by the wearable device by using one or more sensors;

   determining, by the electronic device, pulmonary function detection data based on the audio data and the physiological data; and

   displaying, by the electronic device, a first graph, wherein the first graph comprises a first standard curve corresponding to first vital capacity standard data and a first graphical marker corresponding to the pulmonary function detection data.

2. The method according to claim 1, wherein the first vital capacity standard data is related to a first gender of a first user and a first height of the first user, and the first standard curve comprises a first curve, a second curve, and a third curve, wherein

   the first curve comprises a curve of a change in vital capacity data of a user group of the first gender at the first height with an age;

   the second curve comprises a curve of a change in vital capacity data of the user group of the first gender at a second height with the age;

   the third curve comprises a curve of a change in vital capacity data of the user group of the first gender at a third height with the age; and

   the second height is greater than the first height, and the third height is less than the first height.

3. The method according to claim 2, wherein the second height comprises an average minimum height of the user group of the first gender, and the third height is an average maximum height of the user group of the first gender.

4. The method according to claim 2, wherein a difference between the first height and the second height is a first threshold, and a difference between the third height and the first height is a second threshold; and the first threshold is the same as or different from the second threshold.

5. The method according to any one of claims 1 to 4, wherein after the displaying, by the electronic device, a first graph, the method further comprises:

   receiving, by the electronic device, a first operation performed by a user;

   obtaining, by the electronic device, second vital capacity standard data in response to the first operation; and

   displaying, by the electronic device, a second graph, wherein the second graph comprises a second standard curve corresponding to the second vital capacity standard data and a second graphical marker corresponding to the pulmonary function detection data, and the second vital capacity standard data is related to the first gender of the first user, wherein

   the second standard curve comprises a plurality of curves of changes in vital capacity data of the user group of the first gender at different heights with the age.

6. The method according to any one of claims 2 to 5, wherein before the displaying, by the electronic device, a first graph, the method further comprises:

   displaying, by the electronic device, a first user interface, wherein the first user interface further comprises first prompt information, the first prompt information comprises identifiers of a plurality of users, and the identifiers of the plurality of users comprise an identifier of the first user;

   receiving, by the electronic device, a first selection operation performed by the user on the identifier of the first user in the first user interface; and

   obtaining, by the electronic device, the first vital capacity standard data after receiving the selection operation performed on the identifier of the first user.

7. The method according to claim 6, wherein after the determining, by the electronic device, pulmonary function detection data based on the audio data and the physiological data, the method further comprises:
storing, by the electronic device, the pulmonary function detection data in a storage area corresponding to the first user, wherein different users correspond to different storage areas.

8. The method according to claim 6 or 7, wherein the first user interface further comprises a first control, and before the receiving, by an electronic device, audio data and physiological data that are sent by a wearable device, the method further comprises:

receiving, by the electronic device, a second operation performed by the user on the first control; and
sending, by the electronic device, a first message to the wearable device in response to the second operation, wherein the first message indicates the wearable device to collect the audio data and the physiological data.

9. The method according to any one of claims 2 to 8, wherein after the displaying, by the electronic device, a first graph, the method further comprises:

displaying, by the electronic device, a second user interface, wherein a second control is displayed in the second user interface;
receiving, by the electronic device, a third operation performed by the user on the second control; and
displaying, by the electronic device, a third graph in response to the third operation, wherein the third graph comprises pulmonary function detection data in a first time period.

10. The method according to claim 9, wherein the second user interface further comprises second prompt information, the second prompt information comprises the identifiers of the plurality of users, and the identifiers of the plurality of users comprise the identifier of the first user; and
before the displaying, by the electronic device, a third graph, the method further comprises:

receiving, by the electronic device, a second selection operation performed by the user on the identifier of the first user in the second user interface; and
obtaining, by the electronic device, pulmonary function detection data of the first user in the first time period based on the identifier of the first user in response to the second selection operation.

11. The method according to any one of claims 1 to 10, wherein the pulmonary function detection data comprises any one or more of the following: a vital capacity, a forced expiratory volume in 1 second/forced vital capacity ratio, a forced expiratory volume in 1 second, peak expiratory flow, peak inspiratory flow, maximal expiratory pressure, maximal inspiratory pressure, maximal voluntary ventilation, and maximal mid-expiratory flow, wherein

the forced expiratory volume in 1 second/forced vital capacity ratio indicates a percentage of an amount of air exhaled in a first second to a total amount of air exhaled in effective duration;
the forced expiratory volume in 1 second indicates a total amount of air exhaled in the first second;
the peak expiratory flow indicates an instantaneous maximum value of exhaled air flow in a maximum forced exhalation process;
the peak inspiratory flow indicates an instantaneous maximum value of inhaled air flow in a maximum forced inhalation process;
the maximal expiratory pressure indicates a maximum value of oral pressure capable of being generated in the maximum forced exhalation process;
the maximal inspiratory pressure indicates a maximum value of oral pressure capable of being generated in the maximum forced inhalation process;
the maximal voluntary ventilation indicates maximum ventilation in 1 minute; and
the maximal mid-expiratory flow indicates a mean expiratory flow rate when 25% to 75% of the vital capacity is exhaled forcibly.

12. The method according to any one of claims 1 to 11, wherein the audio data comprises any one or more of the following: a cough sound, a blow sound, and a speaking sound; and
the physiological data comprises any one or more of the following: a heart rate, heart rate variability, a respiratory rate, and blood oxygen.

13. A health data display system, wherein the system comprises a wearable device and an electronic device, the wearable device establishes a communication connection to the electronic device, and the wearable device comprises a microphone and one or more motion sensors, wherein

the wearable device is configured to: collect audio data by using the microphone, and collect physiological data by using the one or more sensors;

the wearable device is further configured to send the audio data and the physiological data to the electronic device;

the electronic device is configured to determine pulmonary function detection data based on the audio data and the physiological data; and

the electronic device is further configured to display a first graph, wherein the first graph comprises a first standard curve corresponding to first vital capacity standard data and a first graphical marker corresponding to the pulmonary function detection data.

14. The system according to claim 13, wherein the first vital capacity standard data is related to a first gender of a first user and a first height of the first user, and the first standard curve comprises a first curve, a second curve, and a third curve, wherein

the first curve comprises a curve of a change in vital capacity data of a user group of the first gender at the first height with an age;

the second curve comprises a curve of a change in vital capacity data of the user group of the first gender at a second height with the age;

the third curve comprises a curve of a change in vital capacity data of the user group of the first gender at a third height with the age; and

the second height is greater than the first height, and the third height is less than the first height.

15. The system according to claim 14, wherein the second height comprises an average minimum height of the user group of the first gender, and the third height is an average maximum height of the user group of the first gender.

16. The system according to claim 14, wherein a difference between the first height and the second height is a first threshold, and a difference between the third height and the first height is a second threshold; and

the first threshold is the same as or different from the second threshold.

17. The system according to any one of claims 13 to 16, wherein the electronic device is further configured to:

receive a first operation performed by a user;

obtain second vital capacity standard data in response to the first operation; and

display a second graph, wherein the second graph comprises a second standard curve corresponding to the second vital capacity standard data and a second graphical marker corresponding to the pulmonary function detection data, and the second vital capacity standard data is related to the first gender of the first user, wherein the second standard curve comprises a plurality of curves of changes in vital capacity data of the user group of the first gender at different heights with the age.

18. The system according to any one of claims 14 to 17, wherein the electronic device is further configured to:

display a first user interface, wherein the first user interface further comprises first prompt information, the first prompt information comprises identifiers of a plurality of users, and the identifiers of the plurality of users comprise an identifier of the first user; and

receive a first selection operation performed by the user on the identifier of the first user in the first user interface; and

the electronic device is specifically configured to obtain the first vital capacity standard data after receiving the selection operation performed on the identifier of the first user.

19. The system according to claim 18, wherein the electronic device is further configured to store the pulmonary function detection data in a storage area corresponding to the first user, wherein different users correspond to different storage areas.

20. The system according to claim 18 or 19, wherein the first user interface further comprises a first control, and the electronic device is further configured to:

receive a second operation performed by the user on the first control; and

send a first message to the wearable device in response to the second operation, wherein the first message indicates the wearable device to collect the audio data and the physiological data.

21. The system according to any one of claims 14 to 20, wherein the electronic device is further configured to:

display a second user interface, wherein a second control is displayed in the second user interface;

receive a third operation performed by the user on the second control; and

display a third graph in response to the third operation, wherein the third graph comprises pulmonary function detection data in a first time period.

22. The system according to claim 21, wherein the second user interface further comprises second prompt information, the second prompt information comprises the identifiers of the plurality of users, and the identifiers of the plurality of users comprise the identifier of the first user; and

the electronic device is further configured to:

receive a second selection operation performed by the user on the identifier of the first user in the second user interface; and

obtain pulmonary function detection data of the first user in the first time period based on the identifier of the first user in response to the second selection operation.

23. The method according to any one of claims 13 to 22, wherein the pulmonary function detection data comprises any one or more of the following: a vital capacity, a forced expiratory volume in 1 second/forced vital capacity ratio, a forced expiratory volume in 1 second, peak expiratory flow, peak inspiratory flow, maximal expiratory pressure, maximal inspiratory pressure, maximal voluntary ventilation, and maximal mid-expiratory flow, wherein

the forced expiratory volume in 1 second/forced vital capacity ratio indicates a percentage of an amount of air exhaled in a first second to a total amount of air exhaled in effective duration;

the forced expiratory volume in 1 second indicates a total amount of air exhaled in the first second;

the peak expiratory flow indicates an instantaneous maximum value of exhaled air flow in a maximum forced exhalation process;

the peak inspiratory flow indicates an instantaneous maximum value of inhaled air flow in a maximum forced inhalation process;

the maximal expiratory pressure indicates a maximum value of oral pressure capable of being generated in the maximum forced exhalation process;

the maximal inspiratory pressure indicates a maximum value of oral pressure capable of being generated in the maximum forced inhalation process;

the maximal voluntary ventilation indicates maximum ventilation in 1 minute; and

the maximal mid-expiratory flow indicates a mean expiratory flow rate when 25% to 75% of the vital capacity is exhaled forcibly.

24. The system according to any one of claims 13 to 23, wherein the audio data comprises any one or more of the following: a cough sound, a blow sound, and a speaking sound; and

the physiological data comprises any one or more of the following: a heart rate, heart rate variability, a respiratory rate, and blood oxygen.

25. An electronic device, wherein the electronic device comprises a processor and a memory, the memory is coupled to the processor, the memory is configured to store computer program code, the computer program code comprises computer instructions, and the processor invokes the computer instructions, to perform the method according to any one of claims 1 to 12.

26. A computer-readable storage medium, wherein the computer-readable storage medium is configured to store computer instructions, and when the computer instructions are run on an electronic device, the server is enabled to perform the method according to any one of claims 1 to 12.

27. A computer program product, wherein when the computer program product runs on an electronic device, the electronic device is enabled to perform the method according to any one of claims 1 to 12.

5G · ▯ 100%

08:00

Thursday
November 19

Communication connection

Wearable device 100

Electronic device 200

FIG. 1

EP 4 599 761 A1

Wearable device 100

Processor 101

Memory 102

Display 104

Motor 105

Wireless communication
module 106

Antenna

Microphone 107

Sensor 103

Gyro sensor 1031

Acceleration sensor 1032

Galvanic skin response
sensor 1033

Touch sensor 1034

Bone conduction sensor 1035

Photoelectric sensor 1036

Motion sensor 1037

FIG. 2

Electronic device 200

Antenna 1

Antenna 2

| Mobile communication module 2G/3G/4G/5G [150] | Wireless communication module BT/WLAN/GNSS/NFC/IR/FM [160] |

Speaker [170A]

Receiver [170B]

Audio module [170]

Microphone [170C]

Headset jack [170D]

Displays 1 to N [194]

Cameras 1 to N [193]

Indicator [192]

Motor [191]

Button [190]

Internal memory [121]

SIM card interfaces 1 to N [195]

External memory interface [120]

Processor [110]

USB port [130]

Charging input

Charging management module [140]

Power management module [141]

Battery [142]

Sensor module [180]

Pressure sensor [180A]

Gyro sensor [180B]

Barometric pressure sensor [180C]

Magnetic sensor [180D]

Acceleration sensor [180E]

Distance sensor [180F]

Optical proximity sensor [180G]

Fingerprint sensor [180H]

Temperature sensor [180J]

Touch sensor [180K]

Ambient light sensor [180L]

Bone conduction sensor [180M]

FIG. 3

FIG. 4A

FIG. 4B

Respiratory health research

420

411

**Risk screening**

15:53 April 19, 2023

Pulmonary function

Good

FEV1/FVC ratio: 85.0% Vital capacity: 4.4 liters

COPD risk                                    Low

Lung infection risk                          Low

Result interpretation: No significant abnormalities are found. It is recommended that you maintain a healthy living habit, pay attention to a balanced diet, and maintain a diet rich in dietary fiber

412

Start measurement

FIG. 4C

5G

100%

A microphone and a sensor need to be
enabled for pulmonary function detection.
Do you agree to enable the microphone
and the sensor?

430

OK

Cancel

FIG. 4D

**5G** ⊿ᵢₗₗ ⁎ 100%

440

Data starts to be collected after vibration.

3    2    1

FIG. 4E

5G | ⚜ Bluetooth | 🔋 100%

450

Detecting ...

FIG. 4F

460

**Record a cough sound**

Please record in a quiet environment

30 cm

45°

Tip: 30 cm is about a length of two mobile phones

Please be in a quiet environment. Pose as shown above. Take a deep breath, and then cough. Repeat deep breathing and coughing two times to three times.

413

11s left

Take a deep breath, and then cough hard. Repeat two times to three times. Stop recording after completion.

21:00

FIG. 4G

FIG. 4H

470

Record a cough sound

🔇 Please record in a quiet environment

30 cm

45°

Tip: 30 cm is about a length of two mobile phones

Please be in a quiet environment. Pose as shown above. Take a deep breath, and then cough. Repeat deep breathing and coughing two times to three times.

▶ 413

6s left

Take a deep breath, and then cough hard. Repeat two times to three times. Stop recording after completion.

FIG. 4I

FIG. 4J

← Collect physiological data

480

7s left
Almost done, please wait patiently.

[Respiratory health tip] The age is a risk factor for COPD.
Prevalence of COPD increases with the age.

FIG. 4K

FIG. 4L

FIG. 4M

490

Respiratory health research

4901

FVC (L)

6

5

4

3

2

1

——— 170 cm      ━━━ 185 cm

——— 165 cm      ━━━ 180 cm

——— 160 cm      ━━━ 175 cm

Age

20   30   40   50   60   70   80

Vital capacity: 3.9 L

FEV1/FVC ratio: 60%

4902

Result interpretation: It is recommended that a user carries out appropriate exercise (for example, jogging and swimming) or breathing training that can improve a cardiopulmonary function; pays attention to a situation of the user; and consults the doctor in time if the user feels uncomfortable.

4903

FIG. 4N

4220

**Respiratory health research**

Risk screening

15:53 April 19, 2023

Pulmonary function

Good

FEV1/FVC ratio: 85.0% Vital capacity: 4.4 liters

COPD risk — Low

Lung infection risk — Low

Result interpretation: No significant abnormalities are found. It is recommended that you maintain a healthy living habit, pay attention to a balanced diet, and maintain a diet rich in dietary fiber

Select a test object — 4202

4201

Start measurement — 412

FIG. 4O

4220

← Respiratory health research

Risk screening

15:53 April 19, 2023

Pulmonary function

Good

FEV1/FVC ratio: 85.0% Vital capacity: 4.4 liters

COPD risk                                    Low

Lung infection risk                          Low

Result interpretation: No significant abnormalities are found. It is recommended that you maintain a healthy living habit, pay attention to a balanced diet, and maintain a diet rich in dietary fiber

AAAA

4203

BBBB

Lisa

Lucy

New detection object

FIG. 4P

FIG. 4Q

FVC (L)

Upper limit
Standard
Lower limit

Age

Pulmonary function detection data of Lucy 4204

Vital capacity: 4.2 L

FEV1/FVC ratio: 75%

Result interpretation: It is recommended that a user carries out appropriate exercise (for example, jogging and swimming) or breathing training that can improve a cardiopulmonary function; pays attention to a situation of the user; and consults the doctor in time if the user feels uncomfortable.

FIG. 4R

**Respiratory health research**

4240

4205

### Risk screening

15:53 April 19, 2023

Pulmonary function

Good

FEV1/FVC ratio: 85.0% Vital capacity: 4.4 liters

COPD risk                                      Low

Lung infection risk                            Low

Result interpretation: No significant abnormalities are found. It is recommended that you maintain a healthy living habit, pay attention to a balanced diet, and maintain a diet rich in dietary fiber

Start measurement                              412

FIG. 4S

FIG. 4T

21:00

Respiratory health research  4203

420
4205
4206

AAAA

BBBB

Lisa

Lucy

Risk sc

15:53 Apr

Pulmonary function

FEV1/FVC ratio: 85.0% Vital capacity: 4.4 liters

COPD risk                                    Low

Lung infection risk                          Low

Result interpretation: No significant abnormalities are found. It is recommended that you maintain a healthy living habit, pay attention to a balanced diet, and maintain a diet rich in dietary fiber

Start measurement   412

FIG. 4U

Recent detection data

4260

**FVC (L)**

```
6
5
4
3
2
1
          April  April  April  April  April  April  April    Time
            1      2      3      4      5      6      7
```

Detection data of Lucy in 7 days

4261

| | |
|---|---|
| April 1, 2023 | Vital capacity: 3.8 L |
| | FEV1/FVC ratio: 65% |
| April 2, 2023 | Vital capacity: 3.9 L |
| | FEV1/FVC ratio: 69% |
| April 3, 2023 | Vital capacity: 4.0 L |

FIG. 4V

S501: An electronic device receives audio data and physiological data that are sent by a wearable device, where the audio data is collected by the wearable device by using a microphone, and the physiological data is collected by the wearable device by using one or more sensors

S502: The electronic device determines pulmonary function detection data based on the audio data and the physiological data

S503: The electronic device displays a first graph, where the first graph includes a first standard curve corresponding to first vital capacity standard data and a first graphical marker corresponding to the pulmonary function detection data

FIG. 5

## INTERNATIONAL SEARCH REPORT

<table>
<tr><td>International application No.<br><br>**PCT/CN2024/093354**</td></tr>
</table>

| A. | CLASSIFICATION OF SUBJECT MATTER |
|---|---|

A61B5/091(2006.01)i

According to International Patent Classification (IPC) or to both national classification and IPC

| B. | FIELDS SEARCHED |
|---|---|

Minimum documentation searched (classification system followed by classification symbols)

IPC:A61B5/-

Documentation searched other than minimum documentation to the extent that such documents are included in the fields searched

Electronic data base consulted during the international search (name of data base and, where practicable, search terms used)

CNPAT, CNKI, DWPI, ENTXT, CNTXT: 华为技术有限公司, 李靖, 崔雨琦, 陈文娟, 显示, 呼吸, 肺活量, 功能, 曲线, 图, 函数, 关系, 界面, 身高, 年龄, 性别, 选择, display, respiration, vital, capacity, function, curve, graph, relation, interface, height, age, gender, select

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 115530768 A (HUAWEI TECHNOLOGIES CO., LTD.) 30 December 2022 (2022-12-30) description, paragraphs [0087]-[0346], and figures 1-20 | 1-27 |
| Y | 王迎彬等 (WANG, Yingbin et al.). "13-15岁壮族青少年年龄、身高、体质量与肺活量的相关性 (Correlation between Age, Height, Weight and Vital Capacity in 13 to 15-Year-Old Adolescents of Zhuang Nationality)" 解剖学杂志 (Chinese Journal of Anatomy), Vol. 39, No. 5, 31 December 2016 (2016-12-31), 591-593, 621 text, pages 591-593 | 1-27 |
| A | US 2015126888 A1 (UNIVERSITY OF WASHINGTON THROUGH ITS CENTER FOR COMMERCIALIZATION) 07 May 2015 (2015-05-07) entire document | 1-27 |
| A | KR 102326251 B1 (ST COMPANY CO., LTD. et al.) 01 December 2021 (2021-12-01) entire document | 1-27 |
| A | KR 102099460 B1 (KIM, Byeong Soo) 09 April 2020 (2020-04-09) entire document | 1-27 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 July 2024** | **02 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/093354**

### C. DOCUMENTS CONSIDERED TO BE RELEVANT

| Category* | Citation of document, with indication, where appropriate, of the relevant passages | Relevant to claim No. |
|---|---|---|
| A | CN 115191961 A (GUANGDONG GENIUS TECHNOLOGY CO., LTD.) 18 October 2022 (2022-10-18) entire document | 1-27 |

Form PCT/ISA/210 (second sheet) (July 2022)

**INTERNATIONAL SEARCH REPORT**
Information on patent family members

International application No.

**PCT/CN2024/093354**

| Patent document cited in search report | | | Publication date (day/month/year) | Patent family member(s) | | | Publication date (day/month/year) |
|---|---|---|---|---|---|---|---|
| CN | 115530768 | A | 30 December 2022 | None | | | |
| US | 2015126888 | A1 | 07 May 2015 | EP | 2846690 | A1 | 18 March 2015 |
| | | | | EP | 2846690 | B1 | 28 October 2020 |
| | | | | JP | 2015521063 | A | 27 July 2015 |
| | | | | JP | 6272308 | B2 | 31 January 2018 |
| | | | | IN | 10232014 | A | 07 August 2015 |
| | | | | WO | 2013170131 | A1 | 14 November 2013 |
| | | | | US | 10028675 | B2 | 24 July 2018 |
| | | | | CA | 2872785 | A1 | 14 November 2013 |
| | | | | CA | 2872785 | C | 29 June 2021 |
| KR | 102326251 | B1 | 01 December 2021 | None | | | |
| KR | 102099460 | B1 | 09 April 2020 | None | | | |
| CN | 115191961 | A | 18 October 2022 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310567670